(19) Europäisches Patentamt
European Patent Office
Office européen des brevets

(11) Numéro de publication : **0 462 884 B1**

(12) # FASCICULE DE BREVET EUROPEEN

(45) Date de publication du fascicule du brevet : **16.06.93 Bulletin 93/24**

(51) Int. Cl.$^5$ : **C07K 5/08,** A61K 37/43

(21) Numéro de dépôt : **91401624.1**

(22) Date de dépôt : **18.06.91**

(54) **Dérivés de TRH, leur procédé de préparation et les compositions pharmaceutiques qui les contiennent.**

(30) Priorité : **18.06.90 FR 9007559**

(43) Date de publication de la demande : **27.12.91 Bulletin 91/52**

(45) Mention de la délivrance du brevet : **16.06.93 Bulletin 93/24**

(84) Etats contractants désignés : **AT BE CH DE DK ES FR GB GR IT LI LU NL SE**

(56) Documents cités : **EP-A- 217 688**

(73) Titulaire : **ADIR ET COMPAGNIE**
**1 rue Carle Hébert**
**F-92415 Courbevoie Cédex (FR)**

(72) Inventeur : **Vincent, Michel**
**8 allée du Prunier Hardy**
**F-92220 Bagneux (FR)**
Inventeur : **Remond, Georges**
**9 avenue des Etats-Unis**
**F-78000 Versailles (FR)**
Inventeur : **Portevin, Bernard**
**6 rue Frédéric Passy**
**F-78990 Elancourt (FR)**
Inventeur : **Herve, Yolande**
**16 rue Eichenberger**
**F-92800 Puteaux (FR)**
Inventeur : **Lepagnol, Jean**
**5 rue de Vlaminck**
**F-78400 Chatou (FR)**

EP 0 462 884 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés peptidiques, leur procédé de préparation et les compositions pharmaceutiques qui les contiennent.

Il est désormais largement reconnu que le système cholinergique exerce une influence bénéfique majeure vis-à-vis des phénomènes mnésiques, tant de mémorisation que de remémoration. De même, il est bien connu que le système noradrénergique est étroitement impliqué dans les facultés de concentration et d'attention. Ces deux systèmes sont déficients lors du vieillissement cérébral et sont très vulnérables lors de maladies dégénératives aigües ou progressives telles que la démence d'Alzheimer et l'accident vasculaire cérébral.

D'autre part, parmi les peptides naturels, la **TRH** (**T**hyrotropin-**R**eleasing-**H**ormone) est capable de faciliter la neurotransmission cholinergique, notamment en favorisant la synthèse du neuromédiateur lorsque celle-ci est rendue déficitaire ou en exacerbant l'effet central d'un agoniste cholinergique.

Toutefois, la **TRH** apportée de façon exogène reste peu active du fait de sa dégradation rapide dans l'organisme.

C'est pourquoi, des analogues tripeptidiques ont été décrits afin d'augmenter de façon importante les effets cholinergiques du peptide naturel.

C'est le cas par exemple des tripeptides décrits dans les brevets FR 2.187.155, 2.287.916, 2.266.515 et 2.345.448 dans lesquels le reste pyroglutamyle est remplacé par un autre reste d'acide hétérocyclique carboxylique qui possèdent des propriétés anti-convulsivantes et anti-dépressives. Enfin, le brevet FR 2.585.709 décrit des peptides dans lesquels le reste prolinamide est remplacé par une structure bicyclique saturée et qui sont capables de stimuler la synthèse d'AMP cyclique au niveau du tissu cérébral. Toutefois, ces dérivés n'ont pratiquement aucune activité lorsqu'ils sont administrés par voie orale.

Les composés de la présente invention se sont montrés particulièrement intéressants par l'intensité de leurs propriétés à faciliter la neurotransmission cholinergique puisque ces propriétés s'exercent jusqu'à des doses 50 fois plus faibles que celles de la **TRH** administrée en référence et de façon beaucoup plus prolongée. Cette intensité d'effet facilitateur est retrouvée de la même manière vis-à-vis de la neurotransmission noradrénergique.

Ainsi, les composés de l'invention peuvent améliorer les performances mnésiques et cognitives grâce à la facilitation simultanée cholinergique et noradrénergique. Ils sont donc utiles pour le traitement des troubles comportementaux et constitutifs associés au vieillissement et aux maladies dégénératives neuronales, aigües ou chroniques, comme par exemple la maladie d'Alzheimer, l'accident vasculaire cérébral, le traumatisme spinal ou la sclérose amyotrophique latérale.

L'invention concerne plus particulièrement de nouveaux dérivés à structure cycloamidique répondant à la formule générale (I) :

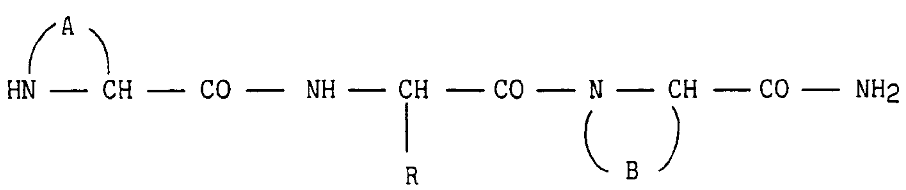

(I)

dans laquelle :

**A** représente avec les atomes d'azote et de carbone avec lesquels il est lié :

- un groupement 2-oxoperhydroazepin-7-yl
- un groupement 2-oxoperhydroazocin-8-yl
- un groupement 2-oxoperhydroazonin-9-yl
- un groupement 2-oxoperhydroazecin-10-yl
- un groupement 2-oxo-2,3,4,7-tétrahydrobenzo[e]azepin-7-yl
- un groupement 2-oxo-2,3,6,7-tétrahydrobenzo[d]azepin-7-yl
- un groupement 2-oxo-2,5,6,7-tétrahydrobenzo[c] azepin-7-yl

**B** représente avec les atomes d'azote et de carbone avec lesquels il est lié une structure polycyclique choisie parmi les structures suivantes :

- 2-azabicyclo [2.2.1] hept-2,3-ylène,
- 2-azabicyclo [2.2.2] oct-2,3-ylène éventuellement substitué en position 1 et 4 par un ou deux groupements alkyles ($C_1$-$C_4$) linéaires ou ramifiés,
- perhydroindol-1,2-ylène,

- perhydroisoindol-2,3-ylène,
- indol-1,2-ylène,
- isoindol-2,3-ylène,
- perhydroquinol-1,2-ylène,
- perhydroisoquinol-2,3-ylène,
- 1,2,3,4-tétrahydroquinol-1,2-ylène,
- 1,2,3,4-tétrahydroisoquinol-2,3-ylène,
- cyclopenta [b] pyrrolidin-1,2-ylène,
- pyrrolidin-1,2-ylène éventuellement substituée par un ou deux groupements alkyles ($C_1$-$C_4$) linéaires ou ramifiés,
- pipéridin-1,2-ylène,
- thiazolidin-3,4-ylène,

**R** représente :

- un atome d'hydrogène
- un groupement alkyle ($C_1$-$C_6$) linéaire ou ramifié éventuellement substitué par un groupement amino ou un groupement guanidino,
- un groupement (imidazol-4-yl) méthyle éventuellement subtitué sur l'un des atomes d'azote par un radical alkyle ($C_1$-$C_4$) linéaire ou ramifié,
- un groupement (pyrazol-3-yl) méthyle,
- un groupement (pyridin-2-yl) méthyle éventuellement substitué par un groupement amino,

leurs énantiomères, diastéréoisomères et épimères, ainsi que leurs sels d'addition à un acide pharmaceutiquement acceptable.

Parmi les acides pharmaceutiquement acceptables, on peut citer à titre non limitatif, les acides chlorhydrique, sulfurique, tartrique, maléïque, fumarique, oxalique, méthane sulfonique, camphorique, etc...

L'invention s'étend aussi au procédé de préparation des dérivés de formule (I) caractérisé en ce que l'on protège la fonction amine d'un amino-acide de formule (II), dont on a éventuellement séparé les isomères par une technique classique de séparation :

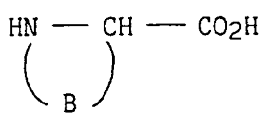

(II)

dans laquelle B a la même signification que dans la formule (I), par un radical protecteur (P) tel que le tert. butoxycarbonyle (tBOC) ou le benzyloxycarbonyle (Z) sous l'action d'un réactif approprié pour conduire à un dérivé de formule (III) :

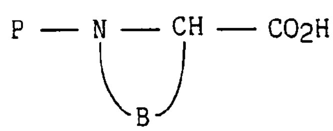

(III)

dans laquelle B et P ont la même signification que précédemment, sur lequel on fait réagir, à une température comprise entre -15 et 0°C, en présence de triéthylamine, le chloroformiate d'éthyle puis l'ammoniaque, pour conduire à un dérivé de formule (IV) :

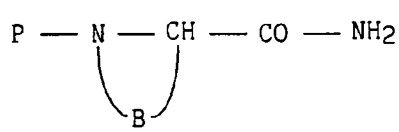

(IV)

dans laquelle B et P ont la même signification que précédemment, que l'on déprotège par un procédé approprié comme par exemple l'action de l'acide chlorhydrique gazeux dans un solvant anhydre tel que le dioxane ou l'acétate d'éthyle dans le cas où P = tBOC ou par hydrogénation catalytique dans le cas où P=Z, pour conduire à un dérivé de formule (V) :

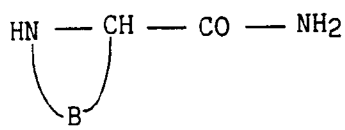

(V)

dans laquelle B a la même signification que dans la formule (I), dont on sépare, si on le souhaite, les isomères par une technique classique de séparation, qui est couplé avec un deuxième amino-acide protégé de formule (VI) selon la technique de couplage peptidique décrite par W.KONIG et R. GEIGER (Ber. 103, 788,1970) :

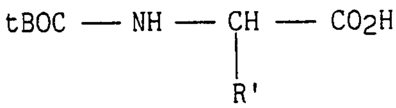

(VI)

dans laquelle R' représente un atome d'hydrogène, un groupement alkyle ($C_1$-$C_6$) linéaire ou ramifié éventuellement substitué par un groupement amino protégé par exemple par un radical benzyloxycarbonyle (Z) ou un groupement guanidino lui-même protégé par exemple par un radical nitro, un groupement (imidazol-4-yl) méthyle éventuellement substitué sur l'un des atomes d'azote par un radical alkyle ($C_1$-$C_4$) linéaire ou ramifié, un groupement (pyrazol-3-yl) méthyle, un groupement (pyridin-2-yl) méthyle éventuellement substitué par un groupement amino protégé par exemple par un radical benzyloxycarbonyle, pour conduire à un dérivé de formule (VII) :

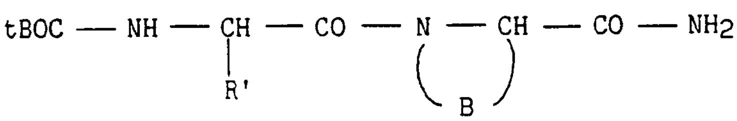

(VII)

dans laquelle R' et B ont la même signification que précédemment, dont on sépare, si on le souhaite, les diastéréoisomères ou énantiomères par une technique classique de séparation, que l'on déprotège ensuite par action de l'acide chlorydrique gazeux dans un solvant anhydre comme par exemple le dioxane ou l'acétate d'éthyle pour conduire à un dérivé de formule (VIII) :

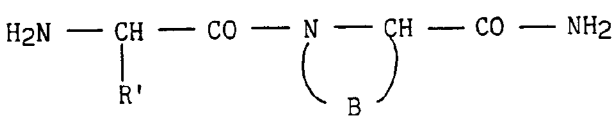

(VIII)

dans laquelle R' et B ont la même signification que précédemment, qui est couplé avec un troisième amino-acide de formule (IX), selon la technique de couplage peptidique décrite par G.ANDERSON et J.ZIMMERMAN (J.A.C.S., 85, 3039, 1963) :

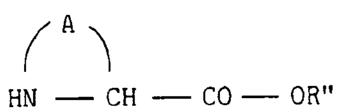

(IX)

dans laquelle R" est un radical succinimide pour conduire :

- ou bien :
  à un dérivé de formule (I) dans le cas où R' est différent d'un groupement alkyle ($C_1$-$C_6$) linéaire ou ramifié substitué par un groupement amino protégé, ou guanidino protégé ou différent d'un groupement

(pyridin-2-yl) méthyle substitué par un groupement amino protégé,

dont on sépare, si on le désire, les isomères selon une technique classique de séparation puis, si nécessaire, que l'on transforme en sel d'addition à un acide pharmaceutiquement acceptable,

- ou bien :

à un dérivé de formule (X)

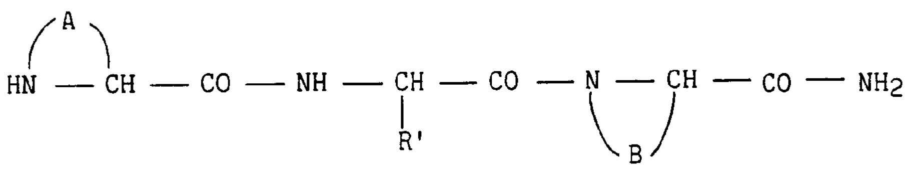

(X)

dans le cas où A et B ont la même signification que dans la formule (I) et R' est un groupement alkyle ($C_1$-$C_6$) linéaire ou ramifié substitué par un groupement amino protégé ou guanidino protégé ou R' est un groupement (pyridin-2-yl) méthyle substitué par un groupement amino protégé,

dont on sépare, si on le désire, les isomères selon une technique classique de séparation et que l'on déprotège par hydrogénation catalytique, par exemple, pour conduire à un dérivé de formule (I), dont on sépare éventuellement les isomères selon une technique classique de séparation puis, si nécessaire que l'on transforme en sel d'addition à un acide pharmaceutiquement acceptable.

Les composés de formule (I) possèdent des propriétés pharmacologiques intéressantes qui se manifestent avec une intensité très supérieure et à des doses bien inférieures à celles des dérivés de l'art antérieur.

Ainsi, dès les doses de 0,1 à 0,3 mg/kg, les dérivés de l'invention facilitent la neurotransmission cholinergique centrale d'une part en restaurant la capture de choline à haute affinité, paramètre limitant de la synthèse d'acétylcholine, lorsque celle-ci est rendue déficiente expérimentalement, et d'autre part en exacerbant les effets cholinergiques centraux d'un agoniste muscarinique.

Aux mêmes faibles doses, ces dérivés facilitent la neurotransmission noradrénergique centrale en s'opposant à la perte des réflexes de retournement et des capacités de vigilance induite par un agoniste $\alpha_2$, la xylazine.

Cette double facilitation permet aux dérivés de l'invention de favoriser à faible dose les capacités mnésiques et les facultés d'attention et motivation.

La présente invention a également pour objet les compositions pharmaceutiques renfermant comme principe actif au moins un composé de formule générale (I) ou un de ses sels d'addition à un acide pharmacologiquement acceptable seul ou en combinaison avec un ou plusieurs excipients ou véhicules inertes, non toxiques.

Parmi, les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 0,05 et 300 mg pour un traitement en 1 ou 3 prises par 24 heures. Les exemples suivants illustrent l'invention et ne la limitent en aucune façon.

Les abréviations utilisées dans les exemples sont les suivantes :

- AZEP à la place de 2-oxoperhydroazepin-7-carbonyl,
- AZOC à la place de 2-oxoperhydroazocin-8-carbonyl,
- AZON à la place de 2-oxoperhydroazonin-9-carbonyl,
- AZEC à la place de 2-oxoperhydroazecin-10-carbonyl,
- 3-oxoBzAZEP à la place de 3-oxo-2,3,4,5-tétrahydro-1H-2-benzazepin-1-carbonyl dont la formule développée est la suivante :

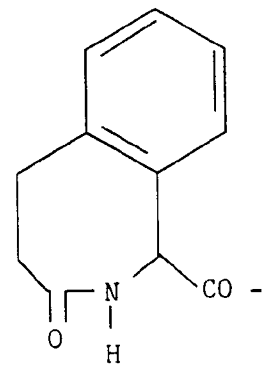

- 1-oxoBzAZEP à la place de 1-oxo-2,3,4,5-tétrahydro-1H-2-benzazepin-3-carbonyl dont la formule développée est la suivante :

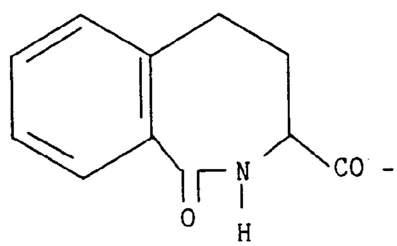

- 2-oxoBzAZEP à la place de 2-oxo-2,3,4,5-tétrahydro-1H-3-benzazepin-4-carbonyl dont la formule développée est la suivante :

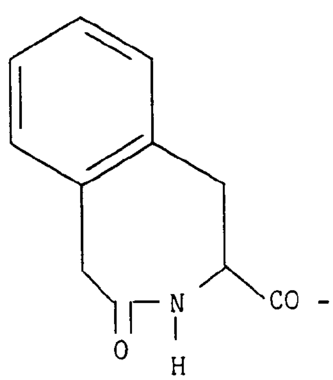

- ($N^{\tau}$-Me)His à la place de 1-méthylhistidyle dont la formule développée est la suivante :

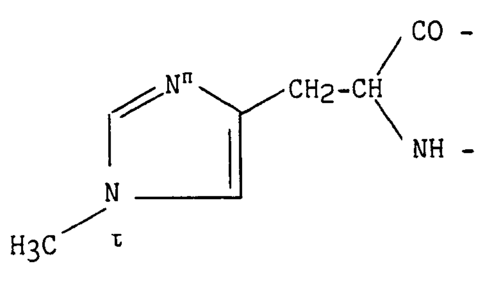

- ($N^{\pi}$-Me)His à la place de 3-méthylhistidyle dont la formule développée est la suivante :

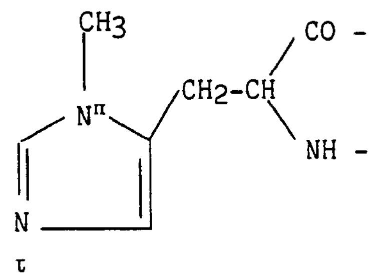

- His à la place de histidyle,
- Leu à la place de leucyle,
- Lys à la place de lysyle,
- Arg à la place de arginyle,
- Gly à la place de glycyle,
- Pyra à la place de (pyrazol-3-yl)alanyle,
- AmPyri à la place de (4-aminopyridin-2-yl)alanyle,
- ABH à la place de 2-azabicyclo [2.2.1.] heptane-3-carbonyl,
- Pro à la place de prolyle,
- BOC à la place de butoxycarbonyle,
- Z à la place de benzyloxycarbonyle,
- ABO à la place de 2-azabicyclo [2.2.2] octane-3-carbonyl,
- PHI à la place de perhydroindole-2-carbonyl,
- THIQ à la place de 1,2,3,4-tetrahydroisoquinoléïne-3-carbonyl,
- ThiaPro à la place de 4-thiaprolyle,
- $Lys_{/z}$ à la place de ε-N-benzyloxycarbonyle-lysyle,
- $Arg/NO_2$ à la place de N-nitro-arginyle.

**EXEMPLE 1 : AZEP-(S)($N^{\tau}$-Me)His-(1S,3S,4R)ABH-$NH_2$"isomère A"**

**Stade A : Ester activé de N-hydroxysuccinimide de (R,S)AZEP-OH**

20 mmoles de AZEP-OH obtenu par saponification selon la méthode décrite par E. PERROTI et coll. (Ann. Chim., Rome, 56, (11), 1358, 1966) de l'ester éthylique correspondant lui-même obtenu selon la méthode décrite par C.J. LU et F.F. BLICKE (C.A. 52, 11086f) sont placées dans un tricol de 500 $cm^3$ muni d'un thermomètre, et d'une garde à chlorure de calcium et contenant 150 $cm^3$ de tétrahydrofurane anhydre.

L'ensemble est refroidi dans de l'eau glacée. 20 mmoles de N-hydroxysuccinimide dissoutes dans 100 $cm^3$ de tétrahydrofurane anhydre sont alors additionnées sous agitation puis 20 mmoles de dicyclohexylcarbodiimide. L'agitation est maintenue 18 heures en laissant l'ensemble revenir à température ambiante. Après filtration de la dicyclohexylurée formée, le produit attendu est obtenu par évaporation du filtrat.

Rendement : 99 %

**Stade B : (R,S)AZEP-(S)($N^{\tau}$-Me)His-(1S,3S,4R)ABH-$NH_2$**

En utilisant la méthode de couplage peptidique décrite par G.W. ANDERSON et J.E. ZIMMERMAN (J.A.C.S., 85, 3039, 1963), on fait réagir 20 mmoles de (S)($N^{\tau}$-Me)His-(1S,3S,4R)ABH-$NH_2$, dichlorhydrate décrit dans la demande de brevet français 89.08672 avec 20 mmoles du composé obtenu au stade A.

Après traitement usuel et purification par chromatographie sur gel de silice en utilisant comme éluant un mélange dichlorométhane/méthanol/ammoniaque dans les proportions 90/10/1, on obtient le produit attendu.

Rendement : 78 %

**Stade C : AZEP-(S)($N^{\tau}$-Me)His-(1S,3S,4R)ABH-$NH_2$ "isomère A"**

Le mélange d'isomères obtenu au stade précédent est séparé par CLHP préparative sur colonne Lichroprep RP-18 en utilisant comme éluant un mélange eau/acétonitrile/acide acétique dans les proportions 97,5/2,5/0,1. Les isomères, nommés "A" et "B" par ordre de sortie de colonne, sont obtenus sous forme d'acétates qui sont transformés en leurs bases par passage sur résine Amberlite IRA 93, puis évaporation et lyophilisation. La pureté isomérique du produit attendu est vérifiée par CLHP.

Pouvoir rotatoire : $[\alpha]_D^{20}$= - 26,8° (c = 1 %, éthanol)
Microanalyse élémentaire :

| | C % | H % | N % |
|---|---|---|---|
| Calculé | 58,59 | 7,02 | 19,52 |
| Trouvé | 58,31 | 7,43 | 19,49 |

**EXEMPLE 2 : AZEP-(S)($N^{\tau}$-Me)His-(1S,3S,4R)ABH-$NH_2$ "isomère B"**

Les stades A, B, et C sont identiques à ceux de l'exemple 1. L'isomère "B" est obtenu au stade C, après élution de l'isomère " A" de l'exemple 1.

Résonance Magnétique Nucléaire du Proton ($D_2O$)

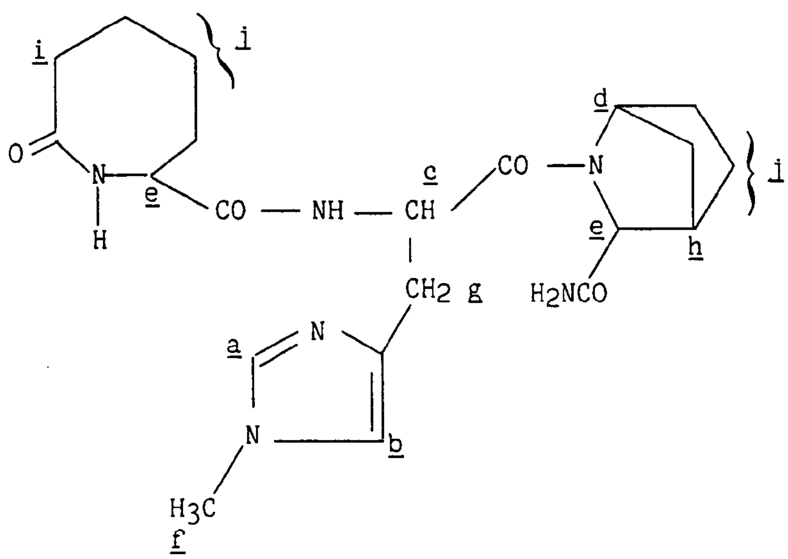

a δ = 7,5 ppm (1H,s)
b δ = 7,0 ppm (1H,s)
c δ = 5,0 ppm (1H,m)
d δ = 4,6 ppm (1H,m)
e δ = 4,2 ppm (2H,m)
f δ = 3,6 ppm (3H,s)
g δ = 3,0 ppm (2H,d)
h δ = 2,8 ppm (1H,m)
i δ = 2,4 ppm (2H,m)
j δ = 1,7 ppm (12H,m)

**EXEMPLE 3 : AZOC-(S)($N^{\tau}$-Me)His-(1S,3S,4R)ABH-$NH_2$ "isomère A"**

**Stade A : Ester activé de N-hydroxy succinimide de (R,S) AZOC-OH**

En procédant comme au stade A de l'exemple 1, mais en remplaçant l'ester éthylique de l'AZEP-OH par l'ester éthylique de l'AZOC-OH, obtenu selon la méthode décrite par C.J.LU et F.F. BLICKE (C.A. 52, 11086f), on obtient le produit attendu.
Rendement : 99 %

**Stade B : (R,S)AZOC-(S)($N^{\tau}$-Me)His-(1S,3S,4R)ABH-$NH_2$**

En procédant comme au stade B de l'exemple 1, mais en remplaçant l'ester activé de (R,S)AZEP-OH par

l'ester activé de (R,S)AZOC-OH obtenu au stade précédent, on obtient le produit attendu.

Rendement : 63 %

**Stade C : AZOC-(S)($N^{\tau}$-Me)His-(1S,3S,4R)ABH-$NH_2$ "isomère A"**

La méthode de séparation et de purification des isomères est la même que celle employée au stade C de l'exemple 1, étant entendu que les isomères "A" et "B" sont ainsi nommés par ordre de sortie de colonne.

Microanalyse élémentaire :

| | C % | H % | N % |
|---|---|---|---|
| Calculé | 59,44 | 7,26 | 18,91 |
| Trouvé | 59,38 | 7,39 | 18,72 |

**EXEMPLE 4 : AZOC-(S)($N^{\tau}$-Me)His-(1S,3S,4R)ABH-$NH_2$ "isomère B"**

Les stades A, B et C sont identiques à ceux de l'exemple 3. La méthode de purification de l'isomère "B", obtenu après élution de l'isomère "A", est la même que celle utilisée pour l'isomère "A" de l'exemple 3.

Résonance Magnétique Nucléaire du Proton ($CDCl_3$) :

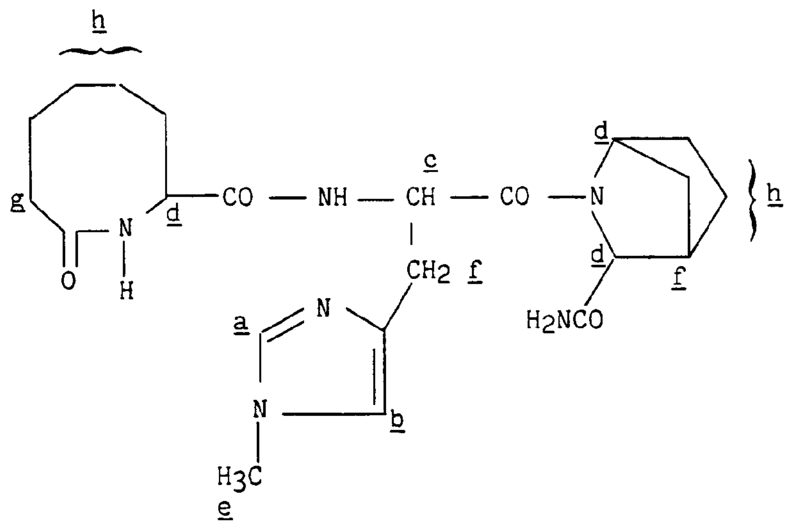

a δ = 7,27 ppm (1H,s)
b δ = 6,65 ppm (1H,s)
c δ = 4,90 ppm (1H,m)
d δ entre 4,6 et 4,0 ppm (3H,m)
e δ = 3,63 ppm (3H,s)
f δ entre 3,22 et 2,85 ppm (3H,m)
g δ entre 2,45 et 2,20 ppm (2H,m)
h δ entre 2,0 et 1,2 ppm (14H,m)

**EXEMPLE 5 : (S)AZEP-(S)His-(S)Pro-$NH_2$**

**Stade A : (R,S)AZEP-(S)His-(S)Pro-$NH_2$**

En procédant comme au stade B de l'exemple 1, mais en remplaçant le (S)($N^{\tau}$-Me)His-(1S,3S,4R)ABH-$NH_2$, dichlorhydrate par le (S)His-(S)Pro-$NH_2$, dichlorhydrate, on obtient le produit attendu.

Rendement : 68 %

**Stade B : (S)AZEP-(S)His(S)Pro-$NH_2$**

La méthode de séparation et de purification des isomères est la même que celle employée au stade C de l'exemple 1. Le solvant d'élution utilisé est un mélange eau/acide acétique dans les proportions 99,8/0,2. Le composé de l'exemple 5 est le premier sorti de la colonne.

Pouvoir rotatoire : $[\alpha]_D^{20}$ = - 36,4° (c = 1 %, éthanol)

Résonance Magnétique Nucléaire du Proton ($CDCl_3$).

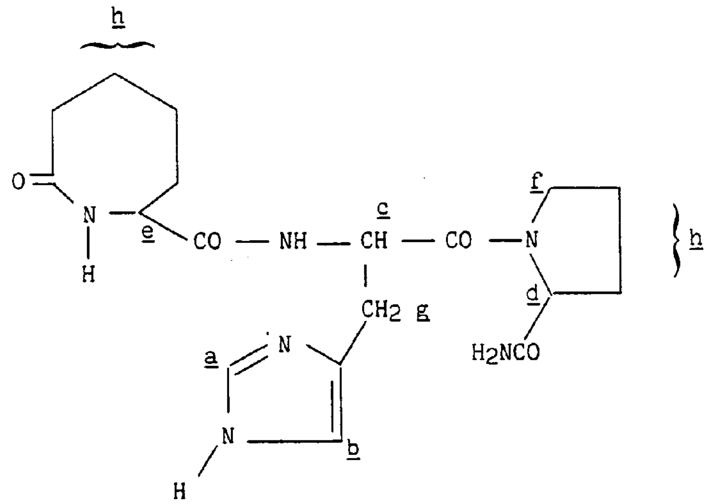

a δ = 7,5 ppm (1H,s)
b δ = 6,9 ppm (1H,s)
c δ = 4,6 ppm (1H,m)
d δ = 4,25 ppm (1H,m)
e δ = 4,05 ppm (1H,m)
f δ entre 3,6 et 3,2 ppm (2H,m)
g δ entre 3,1 et 2,4 ppm (2H,m)
h δ entre 2,4 et 1,2 ppm (12H,m)

**EXEMPLE 5a : (S)AZEP-(S)His-(S)Pro-$NH_2$**

**Stade A : (S)AZEP-OH**

Le produit attendu est obtenu après dérivatisation du (R,S)AZEP-OH par de la (S)(-)α-méthyl-benzylamine, recristallisations du dérivé dans un mélange acétate d'éthyle/méthanol (90/10) puis hydrolyse.

Le pouvoir rotatoire de l'acide 2-aminopimélique obtenu après 5 heures de reflux en milieu chlorhydrique concentré du produit de dérivatisation est comparé à celui de l'acide 2-aminopimélique de configuration connue décrit par R. WADE et Coll. (J.A.C.S., 79, 648-652, 1957).

Pouvoir rotatoire de l'acide 2-(S)-aminopimélique : $[\alpha]_D^{20}$ = + 21,5° (c = 1 %, HCL5N)

Il permet de déduire que l'isomère obtenu de l'AZEP-OH est de configuration S.

Pouvoir rotatoire : $[\alpha]_D^{20}$ = + 9,24° (c = 1 %, éthanol)

**Stade B : (S)AZEP-(S)His-(S)Pro-$NH_2$**

Le produit attendu est obtenu comme au stade A de l'exemple 5 mais en remplaçant le (R,S)AZEP-OH par le (S)AZEP-OH obtenu au stade précédent.

Les caractéristiques physicochimiques sont celles du composé de l'exemple 5.

**EXEMPLE 6 : (R)AZEP-(S)His-(S)Pro-$NH_2$**

Les stades A et B sont identiques à ceux de l'exemple 5. Le composé de l'exemple 6 est obtenu, après élution du composé de l'exemple 5.

Résonance Magnétique Nucléaire du Proton ($CDCl_3$) :

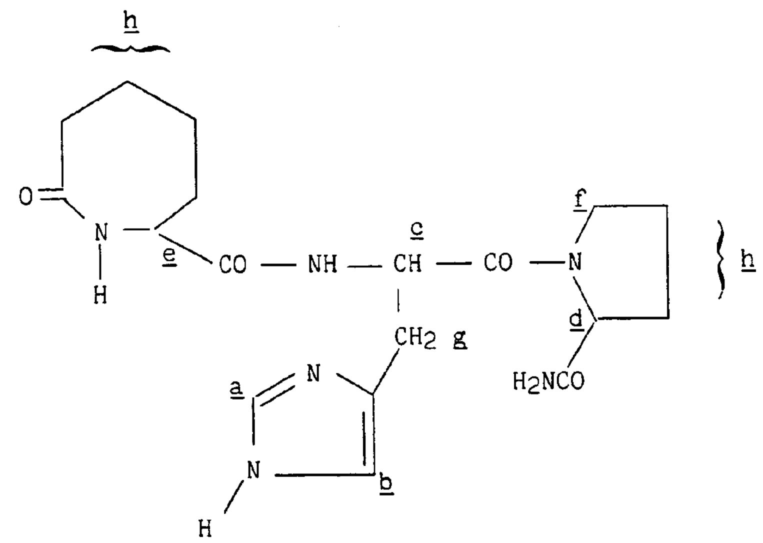

a δ = 7,5 ppm (1H,s)
b δ = 6,9 ppm (1H,s)
c δ = 4,6 ppm (1H,m)
d δ = 4,25 ppm (1H,m)
e δ = 4,05 ppm (1H,m)
f δ entre 3,6 et 3,2 ppm (2H,m)
g δ entre 3,1 et 2,4 ppm (2H,m)
h δ entre 2,4 et 1,2 ppm (12H,m)

**EXEMPLE 7 : AZEP-(S)His-(1S,3S,4R)ABH-$NH_2$ "isomère A"**

**Stade A : (R,S)AZEP-(S)His-(1S,3S,4R)ABH-$NH_2$**

En procédant comme au stade B de l'exemple 1, mais en remplaçant le (S)($N^{\tau}$-Me)His-(1S,3S,4R)ABH-$NH_2$, dichlohydrate par le (S)His-(1S,3S,4R)ABH-$NH_2$, dichlorhydrate décrit dans la demande de brevet français 89.08672, on obtient le produit attendu.

Le solvant d'élution utilisé pour la séparation des isomères est un mélange eau/acide acétique dans les proportions 99,8/0,2.

Rendement :64 %

Résonance Magnétique Nucléaire du Proton ($CDCl_3$) :

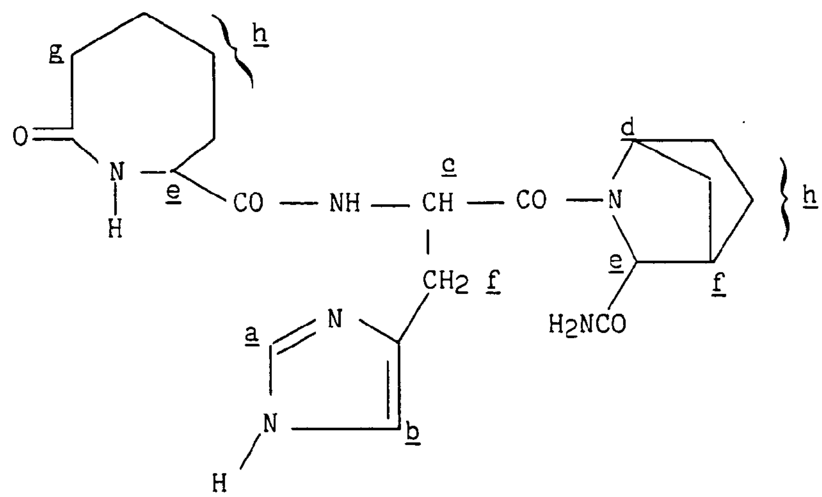

a δ = 7,55 ppm (1H,s)
b δ = 6,85 ppm (1H,s)
c δ = 4,8 ppm (1H,m)
d δ = 4,55 ppm (1H,m)
e δ entre 4,1 et 3,9 ppm (2H,m)
f δ entre 3,1 et 2,6 ppm (3H,m)
g δ entre 2,4 et 2,1 ppm (2H,m)
h δ entre 1,9 et 1,2 ppm (12H,m)

**EXEMPLE 8 : AZEP-(S)His-(1S,3S,4R)ABH-$NH_2$ "isomère B"**

Les stades A et B sont identiques à ceux de l'exemple 7. L'isomère "B" est obtenu, après élution de l'isomère "A" de l'exemple 7.

Microanalyse élémentaire :

| | C % | H % | N % |
|---|---|---|---|
| Calculé | 57,68 | 6,78 | 20,18 |
| Trouvé | 57,99 | 6,57 | 20,40 |

**EXEMPLE 9 : AZOC-(S)His-(S)Pro-$NH_2$ "isomère A"**

En procédant comme au stade B de l'exemple 3, mais en remplaçant le (S)($N^{\tau}$-Me)His-(1S,3S,4R)ABH-$NH_2$, dichlorhydrate par le (S)His-(S)Pro-$NH_2$, dichlorhydrate, on obtient le produit attendu.

Le solvant d'élution utilisé pour la séparation des isomères est un mélange eau/acide acétique dans les proportions 99,8/0,2.

Résonance Magnétique Nucléaire du Proton (DMSO $d_6$) :

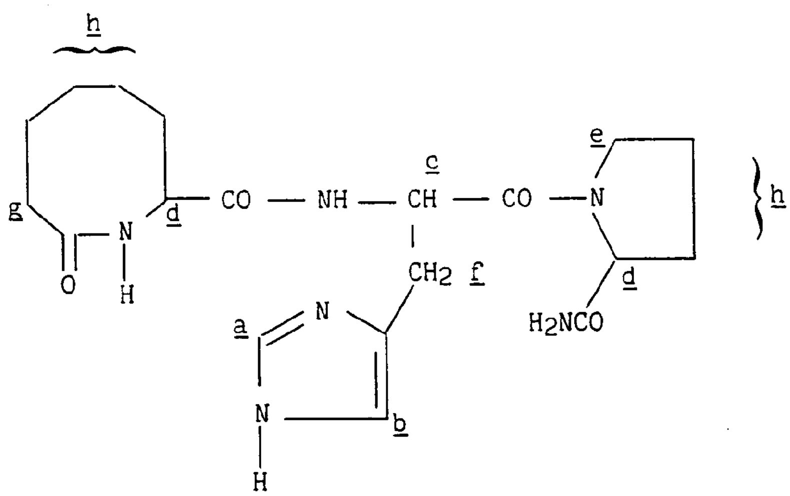

a δ = 7,55 ppm (1H,s)
b δ = 6,9 ppm (1H,s)
c δ = 4,6 ppm (1H,m)
d δ entre 4,4 et 4,1 ppm (2H,m)
e δ entre 3,5 et 3,2 ppm (2H,m)
f δ = 2,9 ppm (2H,m)
g δ = 2,05 ppm (2H,m)
h δ entre 1,9 et 1,1 ppm (12H,m)

**EXEMPLE 10 : AZOC-(S)His-(S)Pro-$NH_2$ "isomère B"**

L'isomère "B" est obtenu, après élution de l'isomère "A" de l'exemple 9.

Résonance Magnétique Nucléaire du Proton (DMSO $d_6$) :

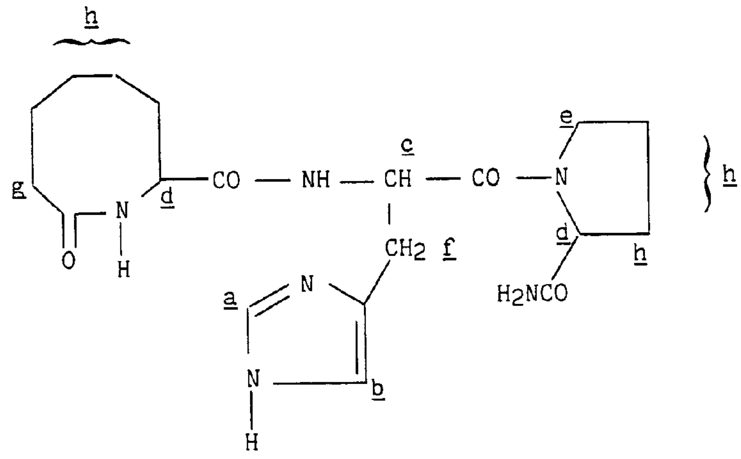

a δ = 7,55 ppm (1H,s)
b δ = 6,9 ppm (1H,s)
c δ = 4,6 ppm (1H,m)
d δ entre 4,4 et 4,1 ppm (2H,m)
e δ entre 3,5 et 3,2 ppm (2H,m)
f δ = 2,9 ppm (2H,m)
g δ = 2,05 ppm (2H,m)
h δ entre 1,9 et 1,1 ppm (12H,m)

**EXEMPLE 11 : AZOC-(S)His-(1S,3S,4R)ABH-$NH_2$ "isomère A"**

En procédant comme dans l'exemple 3 mais en remplaçant au stade B le (S)($N^{\tau}$-Me)His-(1S,3S,4R)ABH-$NH_2$ dichlorhydrate, par le (S)His-(1S,3S,4R)ABH-$NH_2$ dichlorhydrate, on obtient le produit attendu. Le solvant d'élution utilisé pour la séparation des isomères est un mélange eau/acide acétique dans les proportions 99,8/0,2.

Résonance Magnétique Nucléaire du Proton (DMSO $d_6$) :

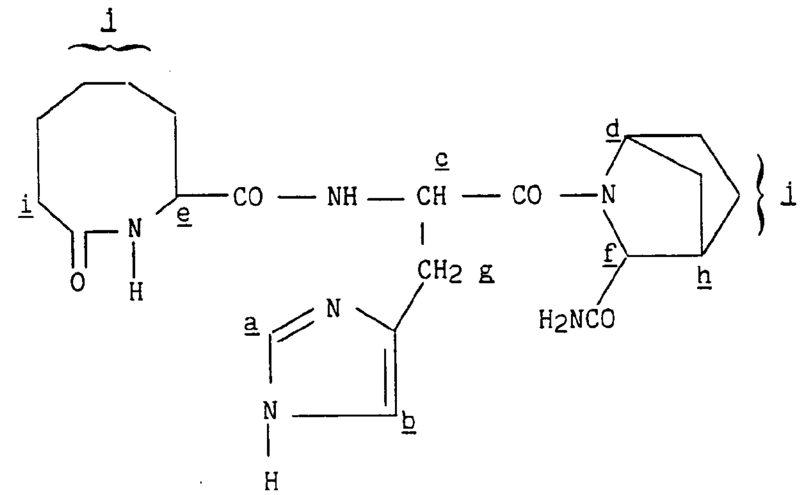

a δ = 7,5 ppm (1H,s)
b δ = 6,9 ppm (1H,s)
c δ = 4,8 ppm (1H,m)
d δ = 4,5 ppm (1H,m)
e δ = 4,3 ppm (1H,m)
f δ = 4,05 ppm (1H,m)
g δ entre 3,1 et 2,8 ppm (2H,m)
h δ = 2,7 ppm (1H,m)
i δ entre 2,45 et 2,1 ppm (2H,m)
j δ entre 1,9 et 1,1 ppm (14H,m)

**EXEMPLE 12 : AZOC-(S)His-(1S,3S,4R)ABH-$NH_2$ "isomère B"**

L'isomère "B" est obtenu, après élution de l'isomère "A" de l'exemple 11.

Résonance Magnétique Nucléaire du Proton (DMSO $d_6$) :

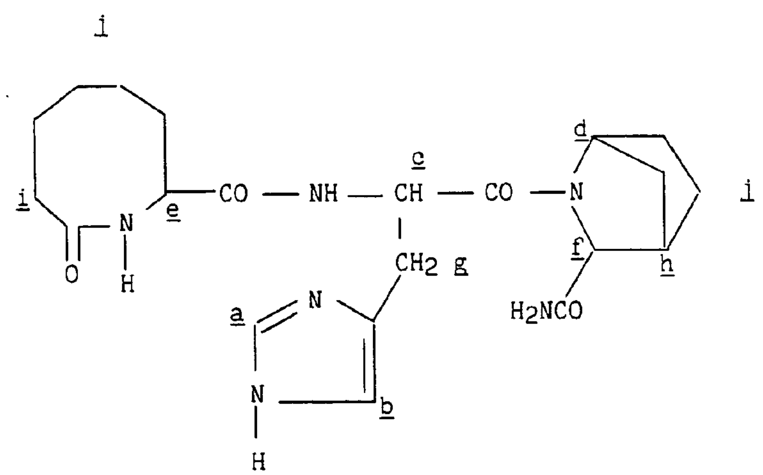

a δ = 7,45 ppm (1H,s)
b δ = 6,8 ppm (1H,s)
c δ = 4,8 ppm (1H,m)
d δ = 4,5 ppm (1H,m)
e δ = 4,3 ppm (1H,m)
f δ = 4,00 ppm (1H,m)
g δ entre 3,1 et 2,7 ppm (2H,m)
h δ = 2,65 ppm (1H,m)
i δ entre 2,45 et 2,1 ppm (2H,m)
j δ entre 1,8 et 1,1 ppm (14H,m)

**EXEMPLE 13 : AZEP-(S)($N^{\tau}$-Me)His-(S)Pro-$NH_2$ "isomère A"**

En procédant comme dans l'exemple 1 mais en remplaçant au stade B le (S)($N^{\tau}$-Me)His-(1S,3S,4R)ABH-$NH_2$, dichlorhydrate par le (S)($N^{\tau}$-Me)His-(S)Pro-$NH_2$, dichlorhydrate, on obtient le produit attendu. (Solvant d'élution : eau/acide acétique : 99,8/0,2)

<u>Résonance Magnétique Nucléaire du Proton</u> (DMSO $d_6$) :

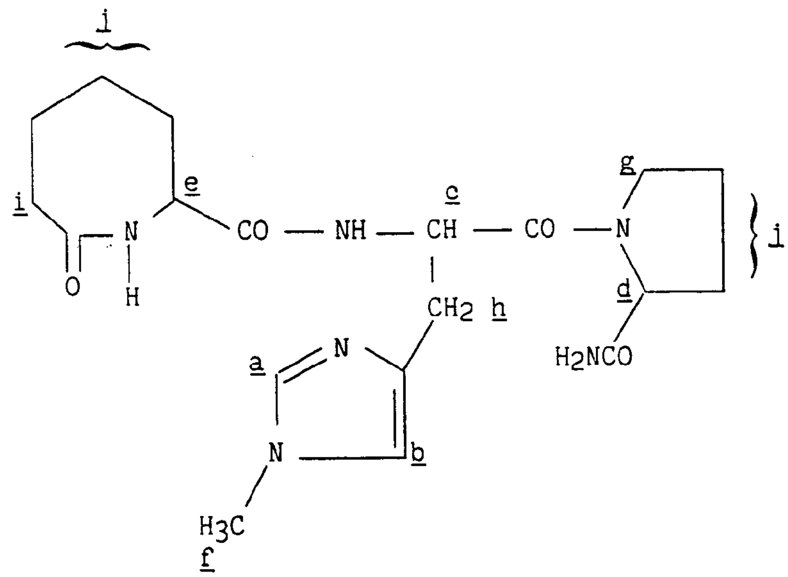

<u>a</u> δ = 7,5 ppm (1H,s)
<u>b</u> δ = 6,9 ppm (1H,s)
<u>c</u> δ entre 4,7 et 4,5 ppm (1H,m)
<u>d</u> δ entre 4,25 et 4,1 ppm (1H,m)
<u>e</u> δ entre 4,1 et 3,9 ppm (1H,m)
<u>f</u> δ = 3,6 ppm (3H,s)
<u>g</u> δ entre 3,5 et 3,2 ppm (2H,s)
<u>h</u> δ entre 3,0 et 2,7 ppm (2H,m)
<u>i</u> δ entre 2,45 et 2,1 ppm (2H,m)
<u>j</u> δ entre 2,1 et 1,3 ppm (10H,m)

**EXEMPLE 14 : AZEP-(S)($N^{\tau}$-Me)His-(S)Pro-$NH_2$ "isomère B"**

L'isomère "B" est obtenu après élution de l'isomère "A" de l'exemple 13.

Résonance Magnétique Nucléaire du Proton (DMSO $d_6$) :

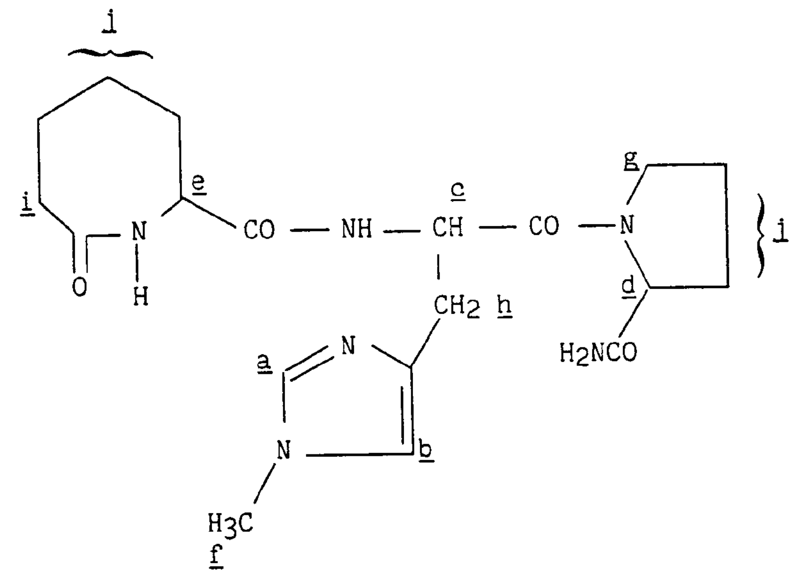

a δ = 7,5 ppm (1H,s)
b δ = 6,9 ppm (1H,s)
c δ entre 4,8 et 4,5 ppm (1H,m)
d δ entre 4,30 et 4,15 ppm (1H,m)
e δ entre 4,0 et 3,9 ppm (1H,m)
f δ = 3,6 ppm (3H,m)
g δ entre 3,4 et 3,2 ppm (2H,m)
h δ entre 3,0 et 2,6 ppm (2H,m)
i δ entre 2,40 et 2,2 ppm (2H,m)
j δ entre 2,1 et 1,3 ppm (10H,m)

**EXEMPLE 15 : AZEP-(S)($N^{\pi}$-Me)His-(S)Pro-$NH_2$ "isomère A"**

En procédant comme dans l'exemple 1 mais en remplaçant au stade B le (S)($N^{\tau}$-Me)His-(1S,3S,4R)ABH-$NH_2$, dichlorhydrate par le (S)($N^{\pi}$-Me)His-(S)Pro-$NH_2$, dichlorhydrate, on obtient le produit attendu.

Rendement : 84 %

Résonance Magnétique Nucléaire du Proton (DMSO $d_6$) :

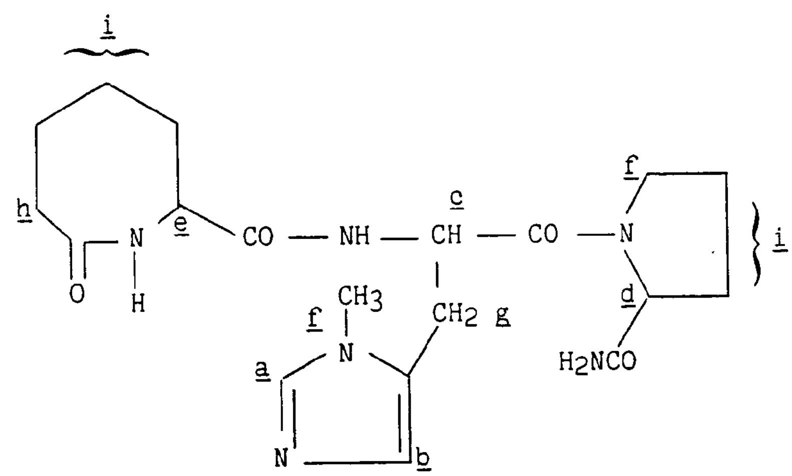

a δ = 7,5 ppm (1H,s)
b δ = 6,5 ppm (1H,s)
c δ = 4,85 ppm (1H,m)
d δ = 4,25 ppm (1H,m)
e δ = 3,95 ppm (1H,m)
f δ = 3,6 ppm (5H,s)
g δ entre 3,0 et 2,6 ppm (2H,m)
h δ = 2,3 ppm (2H,m)
i δ entre 1,9 et 1,2 ppm (10H,m)

**EXEMPLE 16 : AZEP-(S)(N$^{\pi}$-Me)His-(S)Pro-$NH_2$ "isomère B"**

L'isomère "B" est obtenu après élution de l'isomère "A" de l'exemple 15.

Résonance Magnétique Nucléaire du Proton (DMSO $d_6$) :

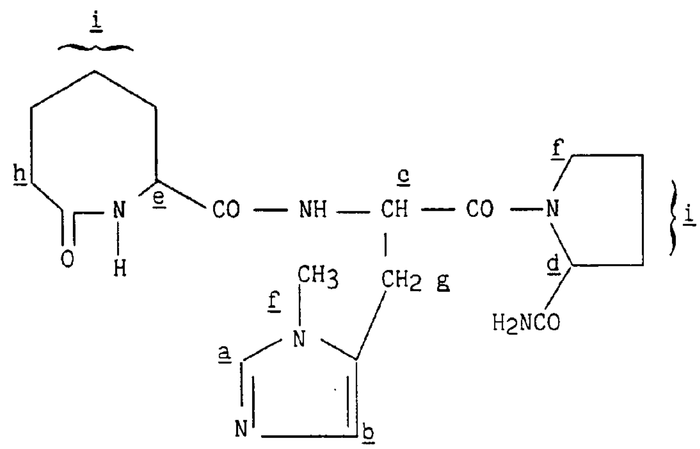

a δ = 7,5 ppm (1H,s)
b δ = 6,5 ppm (1H,s)
c δ = 4,85 ppm (1H,m)
d δ = 4,25 ppm (1H,m)
e δ = 3,95 ppm (1H,m)
f δ = 3,6 ppm (5H,m)
g δ entre 3,0 et 2,6 ppm (2H,m)
h δ = 2,3 ppm (2H,m)
i δ entre 1,9 et 1,2 ppm (10H,m)

**EXEMPLE 17 : AZEP-(S)Leu-(1S,3S,4R)ABH-$NH_2$ "isomère A"**

En procédant comme dans l'exemple 1, mais en remplaçant au stade B le (S)(N$^{\tau}$-Me)His-(1S,3S,4R)ABH-$NH_2$, dichlorhydrate par le (S)Leu-(1S,3S,4R)ABH-$NH_2$, chlorhydrate décrit dans la demande de brevet français 89.08672, on obtient le produit attendu.

Le solvant d'élution utilisé pour la séparation des isomères est un mélange dichlorométhane/méthanol/ammoniaque dans les proportions 90/10/0,5.

Rendement : 87 %

Microanalyse élémentaire :

| | C % | H % | N % |
|---|---|---|---|
| Calculé | 61,20 | 8,22 | 14,27 |
| Trouvé | 61,66 | 7,99 | 14,14 |

**EXEMPLE 18 : AZEP-(S)Leu-(1S,3S,4R)ABH-$NH_2$ "isomère B"**

L'isomère "B" est obtenu après élution de l'isomère "A" de l'exemple 17.

Microanalyse élémentaire :

| | C % | H % | N % |
|---|---|---|---|
| Calculé | 61,20 | 8,22 | 14,27 |
| Trouvé | 61,39 | 8,01 | 14,30 |

**EXEMPLE 19 : AZEP-(S)Leu-(S)Pro-$NH_2$ "isomère A"**

En procédant comme dans l'exemple 1 mais en remplaçant le (S)($N^{\tau}$-Me)His-(1S,3S,4R)ABH-$NH_2$, dichlorhydrate par le (S)Leu-(S)Pro-$NH_2$, chlorhydrate, on obtient le produit attendu.

Le solvant d'élution utilisé pour la séparation des isomères est un mélange dichlorométhane/méthanol/ammoniaque dans les proportions 90/10/0,5.

Rendement : 91 %

Microanalyse élémentaire :

| | C % | H % | N % |
|---|---|---|---|
| Calculé | 59,00 | 8,25 | 15,29 |
| Trouvé | 59,14 | 8,50 | 15,11 |

**EXEMPLE 20 : AZEP-(S)Leu-(S)Pro-$NH_2$ "isomère B"**

L'isomère "B" est obtenu après élution de l'isomère "A" de l'exemple 19.

Microanalyse élémentaire :

| | C % | H % | N % |
|---|---|---|---|
| Calculé | 59,00 | 8,25 | 15,29 |
| Trouvé | 59,33 | 8,35 | 14,79 |

**EXEMPLE 21 : AZEP-(S)Lys-(S)Pro-$NH_2$ "isomère A"**

**Stade A : AZEP-(S)$Lys_{/z}$-(S)Pro-$NH_2$ "isomère A"**

En procédant comme au stade B de l'exemple 1, mais en remplaçant le (S)($N^{\tau}$-Me)His-(1S,3S,4R)ABH-$NH_2$, dichlorhydrate par le (S)$Lys_{/z}$-(S)Pro-$NH_2$, trifluoroacétate, on obtient le produit attendu.

Le solvant d'élution utilisé pour la séparation des isomères est un mélange dichlorométhane/méthanol/ammoniaque dans les proportion 90/10/0,5.

Rendement : 77 %

**Stade B : AZEP-(S)Lys-(S)Pro-$NH_2$ "isomère A"**

La déprotection du composé obtenu au stade A est réalisée par hydrogénolyse dans de l'éthanol en présence de charbon palladié. Après filtration du catalyseur et évaporation de l'éthanol, le produit attendu est obtenu par dissolution dans l'eau puis lyophilisation.

Microanalyse élémentaire :

| | C % | H % | N % |
|---|---|---|---|
| Calculé | 56,67 | 8,19 | 18,36 |
| Trouvé | 56,26 | 7,89 | 17,95 |

**EXEMPLE 22 : AZEP-(S)Lys-(S)Pro-$NH_2$ "isomère B"**

L'isomère "B" est obtenu après élution de l'isomère "A" de l'exemple 21.
Microanalyse élémentaire :

| | C % | H % | N % |
|---|---|---|---|
| Calculé | 56,67 | 8,19 | 18,36 |
| Trouvé | 56,12 | 8,27 | 17,90 |

**EXEMPLE 23 : AZEP-(S)Lys-(1S,3S,4R)ABH-$NH_2$ "isomère A"**

**Stade A : BOC(S)$Lys_{/z}$-(1S,3S,4R)ABH-$NH_2$**

En utilisant la technique de couplage peptidique décrite par W.KONIG et R.GEIGER (Ber, 103, 788, 1970) et le diméthylformamide comme solvant, on obtient à partir de BOC(S)$Lys_{/z}$ et de (1S,3S,4R)ABH-$NH_2$, le produit attendu après purification sur gel de silice (solvant d'élution : dichlorométhane/méthanol : 97/3).
Rendement : 84 %

**Stade B : (S)$Lys_{/z}$-(1S,3S,4R)ABH-$NH_2$, chlorhydrate**

Le composé obtenu au stade A est déprotégé dans de l'acétate d'éthyle chlorhydrique 4N pendant 1 heure à 0°C puis 18 heures à température ambiante.
Le produit attendu est obtenu par filtration du précipité, lavage à l'éther puis séchage.
Rendement : 90 %

**Stade C : AZEP-(S)$Lys_{/z}$-(1S,3S,4R)ABH-$NH_2$ "isomère A"**

En procédant comme au stade A de l'exemple 21 mais en remplaçant le (S)$Lys_{/z}$-(S)Pro-$NH_2$, trifluoroacétate par le (S)$Lys_{/z}$-(1S,3S,4R)ABH-$NH_2$, chlorhydrate obtenu au stade précédent, on obtient le produit attendu.

**Stade D : AZEP-(S)Lys-(1S,3S,4R)ABH-$NH_2$ "isomère A"**

La méthode de déprotection est la même que celle utilisée au stade B de l'exemple 21.

Résonance Magnétique Nucléaire du Proton (DMSO $d_6$) :

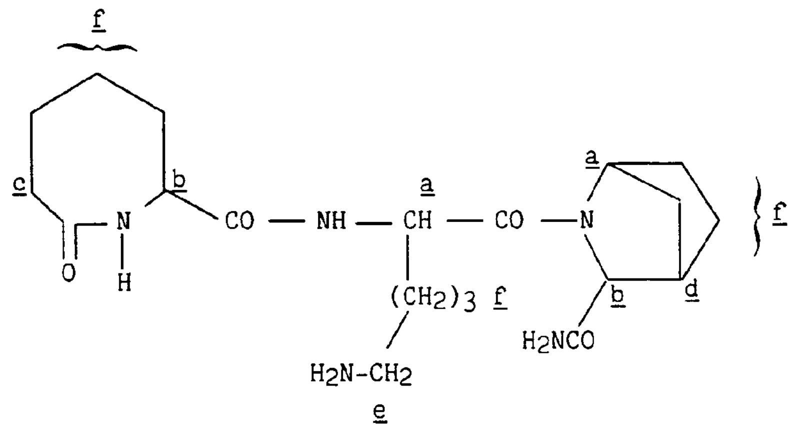

a δ entre 4,6 et 4,4 ppm (2H,m)
b δ entre 4,2 et 4,1 ppm (2H,m)
c δ entre 3,0 et 2,5 ppm (2H,m)
d δ = 2,6 ppm (1H,m)
e δ entre 2,4 et 2,2 ppm (2H,m)
f δ entre 2,0 et 1,2 ppm (18H,m)

**EXEMPLE 24 : AZEP-(S)Lys-(1S,3S,4R)ABH-$NH_2$ "isomère B"**

L'isomère "B" est obtenu après déprotection de l'isomère B élué après l'isomère "A" au stade C de l'exemple 23.

Résonance Magnétique Nucléaire du Proton (DMSO $d_6$) :

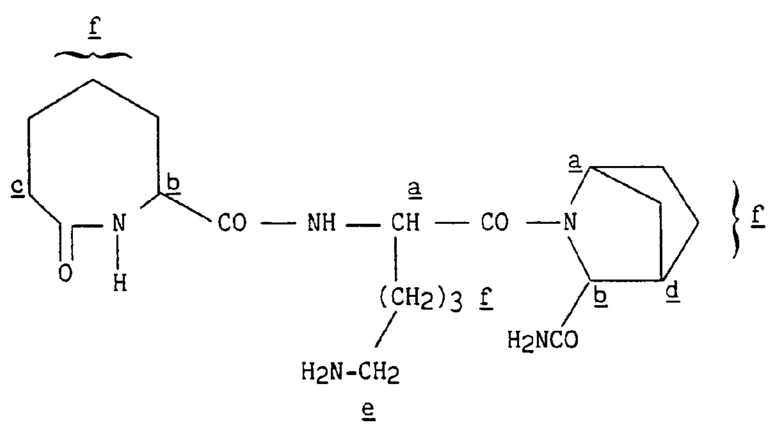

a δ entre 4,6 et 4,4 ppm (2H,m)
b δ entre 4,2 et 4,1 ppm (2H,m)
c δ entre 3,0 et 2,5 ppm (2H,m)
d δ = 2,6 ppm (1H,m)
e δ entre 2,4 et 2,2 ppm (2H,m)
f δ entre 2,0 et 1,2 ppm (18H,m)

**EXEMPLE 25 : AZEP-(S)Arg-(1S,3S,4R)ABH-$NH_2$ "isomère A"**

En procédant comme dans l'exemple 23 mais en remplaçant au stade A le BOC(S)$Lys_{/z}$ par le BOC(S)Arg/$NO_2$, et en effectuant la déprotection du groupement arginyle avant la séparation des isomères "A" et "B", on obtient le produit attendu. Le solvant utilisé pour la séparation des isomères est un mélange acide acétique/eau : 2/1000.

Résonance Magnétique Nucléaire du Proton (DMSO $D_6$)

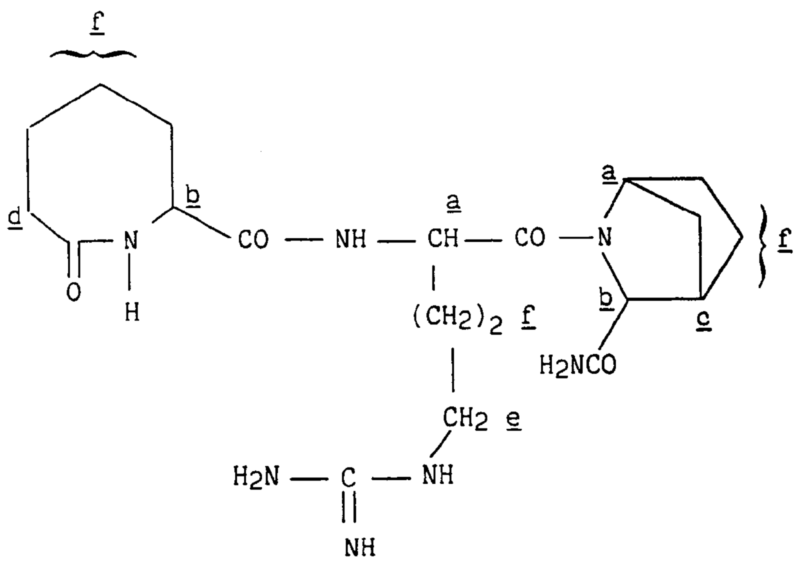

a δ entre 4,6 et 4,4 ppm (2H,m)
b δ entre 4,15 et 4,00 ppm (2H,m)
c δ = 2,65 ppm (1H,m)
d δ = 3,05 ppm (2H,m)
e δ entre 2,5 et 2,1 ppm (2H,m)
f δ entre 2,0 et 1,3 ppm (16H,m)

**EXEMPLE 26 : AZEP-(S)Arg-(1S,3S,4R)ABH-$NH_2$ "isomère B"**

L'isomère "B" est obtenu après élution de l'isomère "A" de l'exemple 25.

Résonance Magnétique Nucléaire du Proton (DMSO $D_6$)

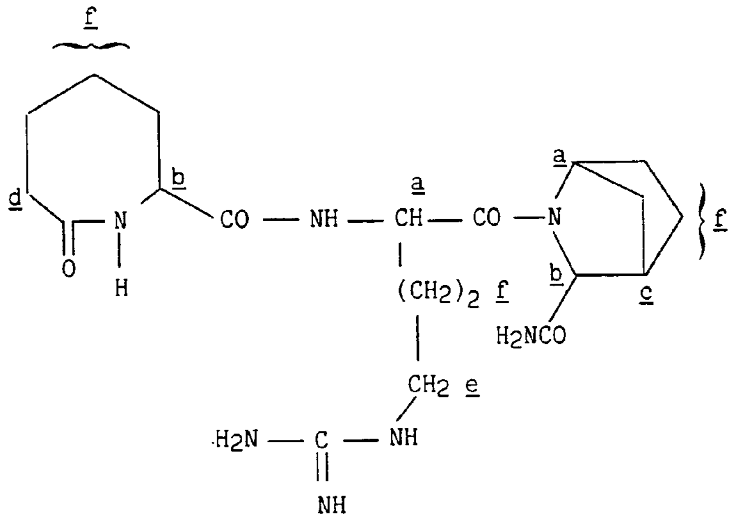

a δ entre 4,6 et 4,4 ppm (2H, m)
b δ entre 4,15 et 4,00 ppm (2H,m)
c δ = 2,65 ppm (1H,m)
d δ = 3,05 ppm (2H,m)
e δ entre 2,5 et 2,1 ppm (2H,m)
f δ entre 2,0 et 1,3 ppm (16H,m)

**EXEMPLE 27 : AZEP-Gly-(1S,3S,4R)ABH-NH$_2$ "isomère A"**

En procédant comme dans l'exemple 23 (stades A, B, C) mais en remplaçant au stade A le BOC(S)Lys$_{/z}$ par le BOC-Gly, on obtient le produit attendu. Le solvant utilisé pour la séparation des isomères est un mélange eau/acétonitrile/diéthylamine : 97/3/0,05.

Résonance Magnétique Nucléaire du Proton (DMSO $D_6$)

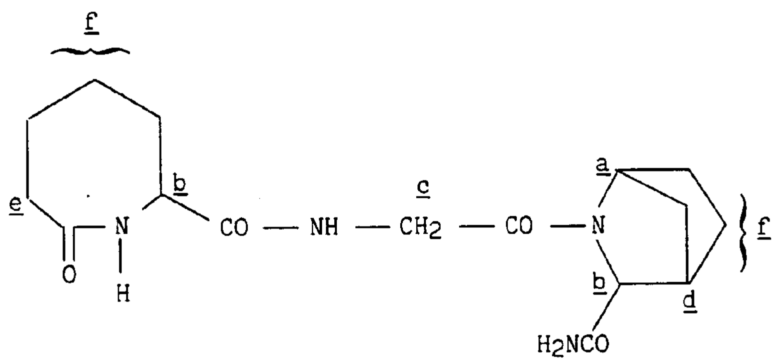

a δ = 4,35 ppm (1H, m)
b δ entre 4,2 et 4,1 ppm (2H,m)
c δ entre 4,2 et 3,9 ppm (2H,Syst.AB)
d δ entre 2,8 et 2,6 ppm (1H,m)
e δ entre 2,5 et 2,2 ppm (2H,m)
f δ entre 2,0 et 1,0 ppm (12H,m)

**EXEMPLE 28 : AZEP-Gly-(1S,3S,4R)ABH-NH$_2$ "isomère B"**

L'isomère "B" est obtenu après élution de l'isomère "A" de l'exemple 27.

Résonance Magnétique Nucléaire du Proton (DMSO $D_6$)

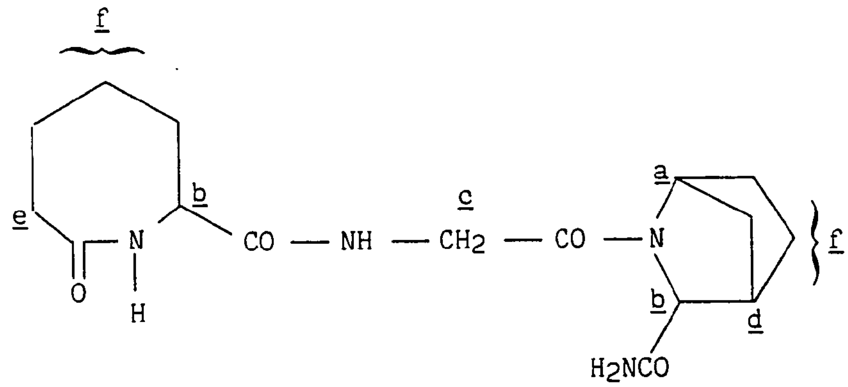

a δ = 4,35 ppm (1H, m)
b δ entre 4,2 et 4,1 ppm (2H,m)
c δ entre 4,2 et 3,9 ppm (2H,Syst.AB)
d δ entre 2,8 et 2,6 ppm (1H,m)
e δ entre 2,5 et 2,2 ppm (2H,m)
f δ entre 2,0 et 1,0 ppm (12H,m)

**EXEMPLE 29 : 3-oxoBzAZEP-(S)($N^{\tau}$-Me)His-(1S,3S,4R)ABH-$NH_2$ "isomère A"**

En procédant comme dans l'exemple 1, mais en remplaçant au stade A l'ester éthylique de (R,S)AZEP-OH par l'ester méthylique de (R,S) 3-oxoBzAZEP-OH obtenu selon la méthode décrite au stade A à partir de méthoxy carbonyl-1 β tétralone décrit par M. PLIENINGER et Coll. (Chem. Ber. 108, 3286,1975), on obtient le produit attendu.

Résonance Magnétique Nucléaire du Proton ($CDCl_3$) :

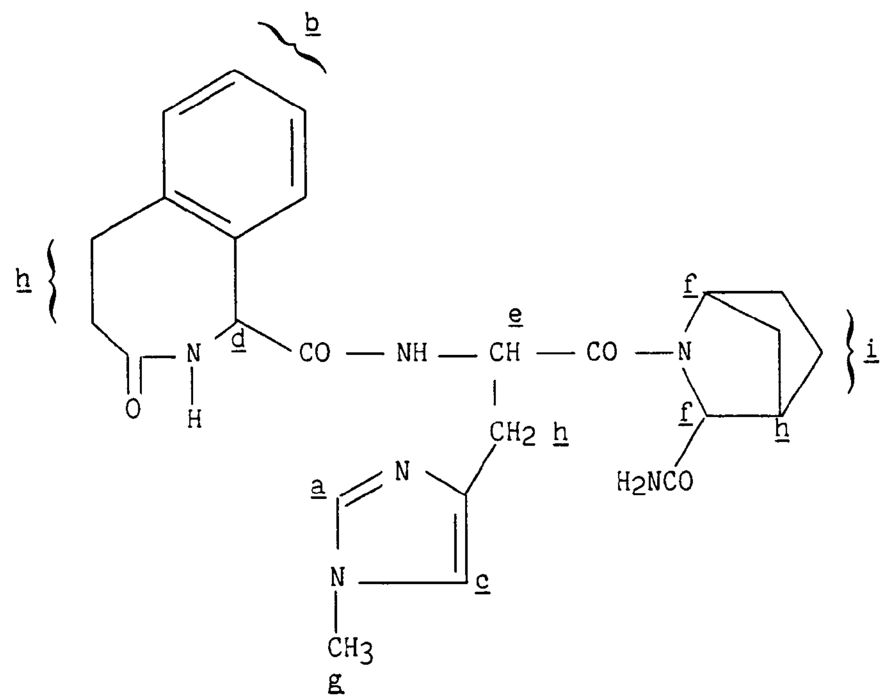

a δ = 7,4 ppm (1H,s)
b δ = 7,2 ppm (4H,m)
c δ = 6,85 ppm (1H,s)

d δ = 5,05 ppm (1H,d)
e δ = 4,8 ppm (1H,m)
f δ entre 4,55 et 3,95 ppm (2H,m)
g δ = 3,55 ppm (3H,s)
h δ entre 3,0 et 2,5 ppm (7H,m)
i δ entre 1,6 et 1,4 ppm (6H,m)

**EXEMPLE 30 : 3-oxoBzAZEP-(S)(Nτ-Me)His-(1S,3S,4R)ABH-$NH_2$ "isomère B"**

L'isomère "B" est obtenu après élution de l'isomère "A" de l'exemple 29.

Résonance Magnétique Nucléaire du Proton ($CDCl_3$) :

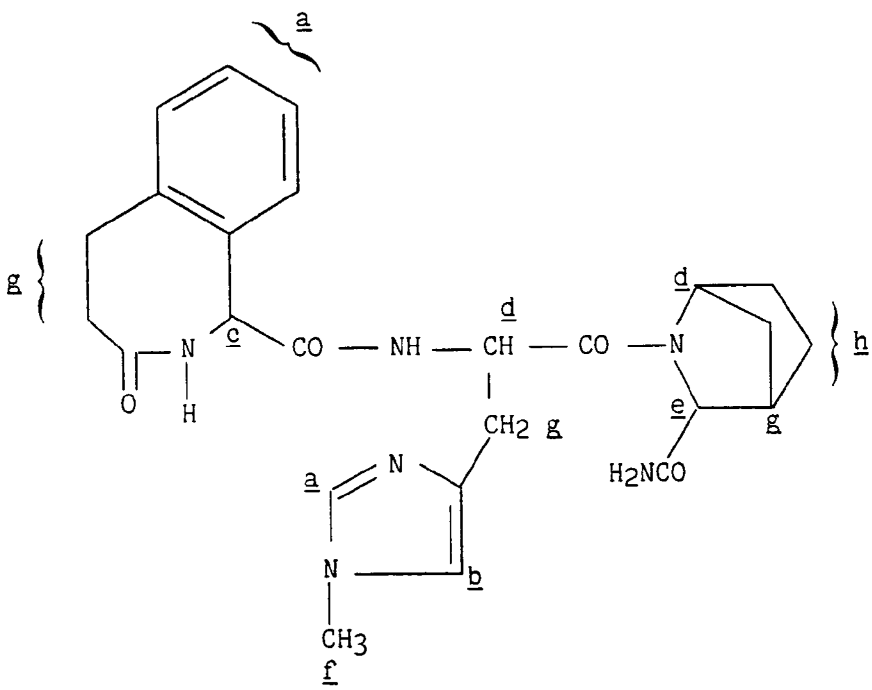

a δ entre 7,4 et 7,2 ppm (5H,m)
b δ = 6,5 ppm (1H,s)
c δ = 5,0 ppm (1H,d)
d δ entre 4,80 et 4,55 ppm (2H,m)
e δ = 3,95 ppm (1H,d)
f δ = 3,45 ppm (3H,s)
g δ entre 3,1 et 2,4 ppm (7H,m)
h δ entre 1,8 et 1,4 ppm (6H,m)

**EXEMPLE 31 : AZEP-Pyra-(1S,3S,4R)ABH-$NH_2$, "isomère A"**

**Stade A : Nα NPyr-DiBoc(R,S)Pyra-OH**

43 mmoles de (R,S)Pyra-OH décrit par R.G. JONES (J.A.C.S., 71, 3994-4000, 1949) sont ajoutées à 100 ml de dioxane et 86 ml de soude 1N. Après refroidissement entre 0 et 5°C, 18,8 g de diterbutyldicarbonate dans 50 ml de dioxane sont ajoutés en 30 minutes. L'agitation est maintenue 20 heures à température ambiante. L'ensemble est neutralisé par addition de 86 ml d'acide chlorhydrique 1N et amené à sec. Le résidu est repris par de l'éthanol. Après filtration du chlorure de sodium et évaporation de l'éthanol, le résidu est finalement repris par 150 ml d'oxyde d'isopropyle. Après filtration et évaporation, on obtient le produit attendu.
Rendement : 98 %

**Stade B : $N^{\alpha}$ $N^{Pyr}$ DiBoc Pyra-(1S,3S,4R)ABH-$NH_2$, "isomère α"**

En utilisant la technique de couplage peptidique décrite par W. KONIG et R. GEIGER (Chem. Ber., 103, 2034, 1970), on fait réagir le produit obtenu au stade précédent avec le (1S,3S,4R)ABH-$NH_2$.
Les isomères α et β sont séparés par chromatographie sur gel de silice.

**Stade C : Pyra-(1S,3S,4R)ABH-$NH_2$, dichlorhydrate, "isomère α"**

Le produit obtenu au stade B est dissous dans du dioxane dans lequel on fait passer un courant d'acide chlorhydrique pendant trente minutes. L'ensemble est maintenu sous agitation 20 heures. Le produit attendu est obtenu par filtration, puis lavé par du dioxane et séché.
Rendement : ≃ 100 %

**Stade D : (R,S)AZEP-Pyra-(1S,3S,4R)ABH-$NH_2$ "isomère α"**

En procédant comme au stade B de l'exemple 1, mais en remplaçant le (S)($N^{\tau}$-Me)His-(1S,3S,4R)ABH-$NH_2$, dichlorhydrate par le produit obtenu au stade précédent, on obtient le produit attendu.
Rendement : 68 %

**Stade E : AZEP-Pyra-(1S,3S,4R)ABH-$NH_2$, "isomère A"**

Le mélange d'isomères obtenu au stade D est séparé par chromatographie liquide préparative sur silice $C_{18}$ en utilisant comme éluant un mélange eau/acétonitrile/acide acétique : 95/5/0,1. Les isomères nommés "A" et "B" par ordre de sortie de colonne sont obtenus sous forme d'acétates qui sont transformés en bases par passage sur résine Amberlite IRA93, puis évaporation et lyophilisation.

Résonance Magnétique Nucléaire du Proton (DMSO $D_6$) :

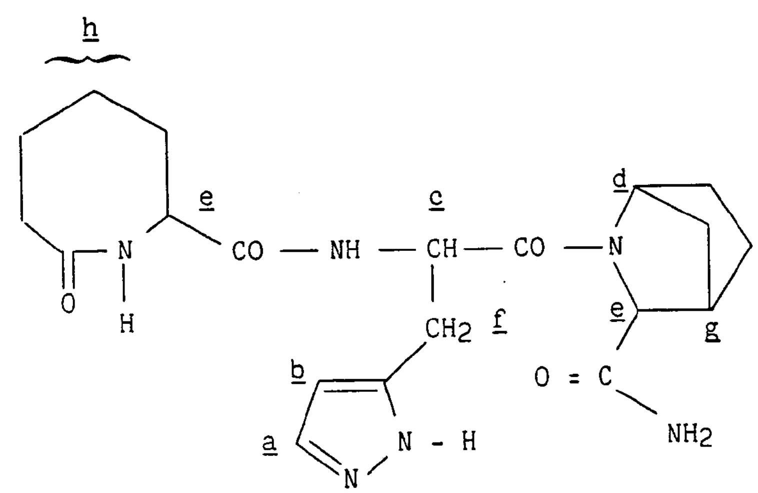

a δ = 7,5 ppm (1H, d)
b δ = 6,1 ppm (1H, d)
c δ = 4,8 ppm (1H, m)
d δ = 4,6 ppm (1H, m)
e δ entre 4,1 et 3,9 ppm (2H, m)
f δ entre 3,2 et 2,7 ppm (2H, m)
g δ = 2,65 ppm (1H, m)
h δ entre 2,4 et 1,3 ppm (14H, m)

**EXEMPLE 32 : AZEP-Pyra-(1S,3S,4R)ABH-$NH_2$, "isomère B"**

Les stades A, B, C, D et E sont identiques à ceux de l'exemple 31. L'isomère "B" est obtenu au stade E, après élution de l'isomère "A" de l'exemple 31.

Microanalyse élémentaire :

| | C % | H % | N % |
|---|---|---|---|
| Calculé | 57,68 | 6,78 | 20,18 |
| Trouvé | 57,54 | 6,61 | 19,91 |

**EXEMPLE 33 : AZEP-Pyra-(1S,3S,4R)ABH-$NH_2$, "isomère C"**

Les stades A, B, C, D et E sont identiques à ceux de l'exemple 31 mais au stade C on utilise l'isomère "β" du $N^{\alpha}$ $N^{Pyr}$ DiBoc-Pyra-(1S,3S,4R)ABH-$NH_2$ à la place de l'isomère "α".

Microanalyse élémentaire :

| | C % | H % | N % |
|---|---|---|---|
| Calculé | 57,68 | 6,78 | 20,18 |
| Trouvé | 58,05 | 6,68 | 19,93 |

**EXEMPLE 34 : AZEP-Pyra-(1S,3S,4R)ABH-$NH_2$, "isomère D"**

Les stades A, B, C, D et E sont identiques à ceux de l'exemple 33. L'isomère "D" est obtenu au stade E, après élution de l'isomère "C" de l'exemple 33.

Microanalyse élémentaire :

| | C % | H % | N % |
|---|---|---|---|
| Calculé | 57,68 | 6,78 | 20,18 |
| Trouvé | 58,00 | 6,58 | 20,23 |

**EXEMPLES 35 à 38 : AZEP-AmPyri-(1S,3S,4R)ABH-$NH_2$, "isomères A, B, C et D"**

**EXEMPLE 39 : 1-oxoBzAZEP-(S)($N^{\tau}$-Me)His-(1S,3S,4R)ABH-$NH_2$, "isomère A"**

En procédant comme dans l'exemple 1, mais en remplaçant au stade A l'ester éthylique de (R,S)AZEP-OH par l'ester éthylique de (R,S)1-oxoBzAZEP-OH obtenu à partir de 2-carbethoxy-α-tétralone décrite par I. UGI et Coll. (Ann., 641, 63-70, 1961), on obtient le produit attendu après séparation des isomères par chromatographie liquide (colonne de silice $C_{18}$, solvant d'élution : eau/acétonitrile/diéthylamine : 90/10/0,5).

Résonance Magnétique Nucléaire du Proton (DMSO $D_6$) :

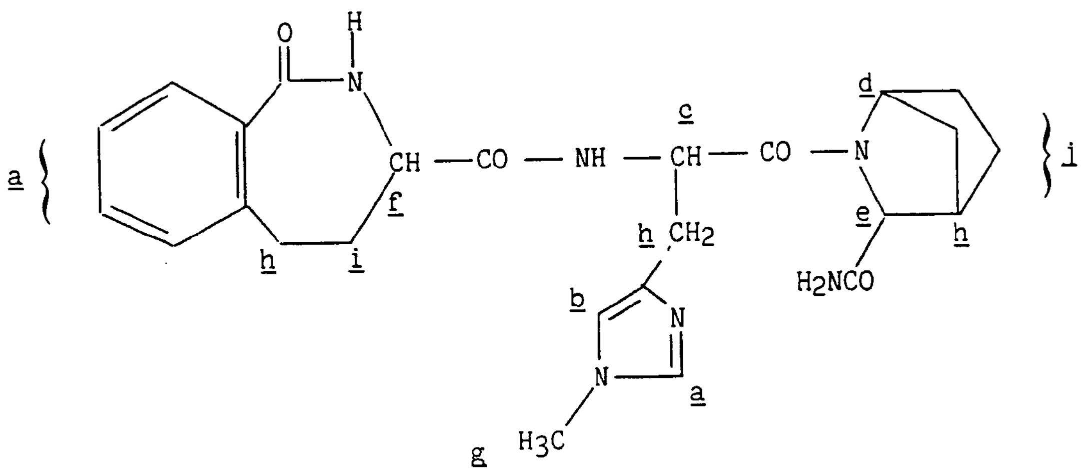

a δ entre 7,5 et 7,2 ppm (5H, m)
b δ = 6,8 ppm (1H, s)
c δ entre 4,75 et 4,6 ppm (1H, m)
d δ = 4,5 ppm (1H, m)
e δ = 3,9 ppm (1H, d)
f δ entre 3,8 et 3,6 ppm (1H, m)
g δ = 3,55 ppm (3H, s)
h δ entre 3,0 et 2,4 ppm (5H, m)
i δ entre 2,3 et 1,8 ppm (2H, m)
j δ entre 1,7 et 1,3 ppm (6H, m)

**EXEMPLE 40 : 1-oxoBzAZEP-(S)($N^{\tau}$-Me)His-(1S,3S,4R)ABH-$NH_2$, "isomère B"**

L'isomère "B" est obtenu après élution de l'isomère "A" de l'exemple 39.

Résonance Magnétique Nucléaire du Proton (DMSO $D_6$) :

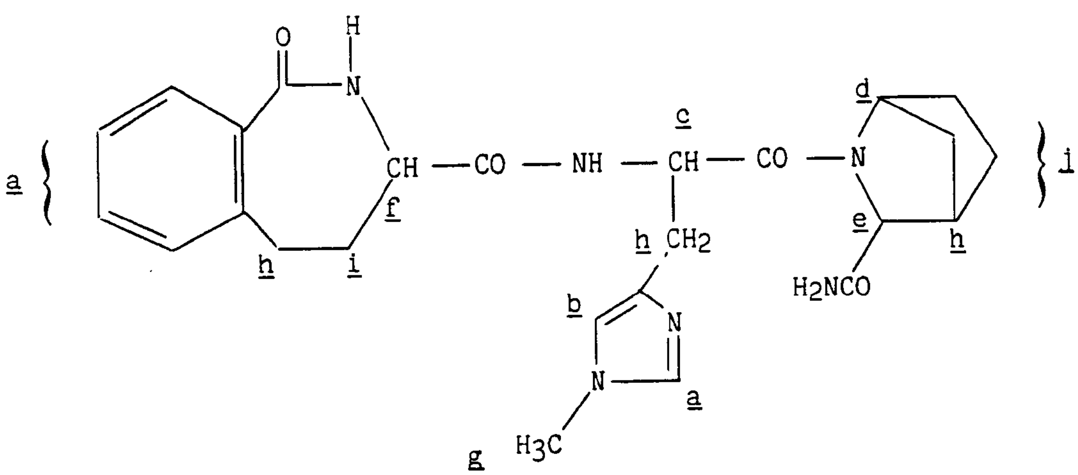

a δ entre 7,5 et 7,2 ppm (5H, m)
b δ = 6,8 ppm (1H, s)
c δ entre 4,75 et 4,6 ppm (1H, m)
d δ = 4,5 ppm (1H, m)
e δ = 3,9 ppm (1H, d)

f δ entre 3,8 et 3,6 ppm (1H, m)
g δ = 3,55 ppm (3H, s)
h δ entre 3,0 et 2,4 ppm (5H, m)
i δ entre 2,3 et 1,8 ppm (2H, m)
j δ entre 1,7 et 1,3 ppm (6H, m)

**EXEMPLES 41 et 42 : 2-oxoBzAZEP-(S)($N^τ$-Me)His-(1S,3S,4R)ABH-$NH_2$**

"isomères A et B"

**EXEMPLES 43 et 44 : AZON-(S)($N^τ$-Me)His-(1S,3S,4R)ABH-$NH_2$**

"isomères A et B"

**EXEMPLE 45 et 46 : AZEC-(S)($N^τ$-Me)His-(1S,3S,4R)ABH-$NH_2$**

"isomères A et B"

**EXEMPLE 47 : AZEP-(S)($N^τ$-Me)His-(S)ABO-$NH_2$, "isomère A"**

Le produit attendu est obtenu en procédant comme dans l'exemple 1 mais en remplaçant le (1S,3S,4R)ABH-$NH_2$ par le (S)ABO-$NH_2$.

Les isomères "A" et "B" sont séparés par chromatographie liquide ( colonne de silice $C_{18}$, solvant d'élution : eau/méthanol/diéthylamine : 80/20/0,1).

Résonance Magnétique Nucléaire du Proton (DMSO $D_6$) :

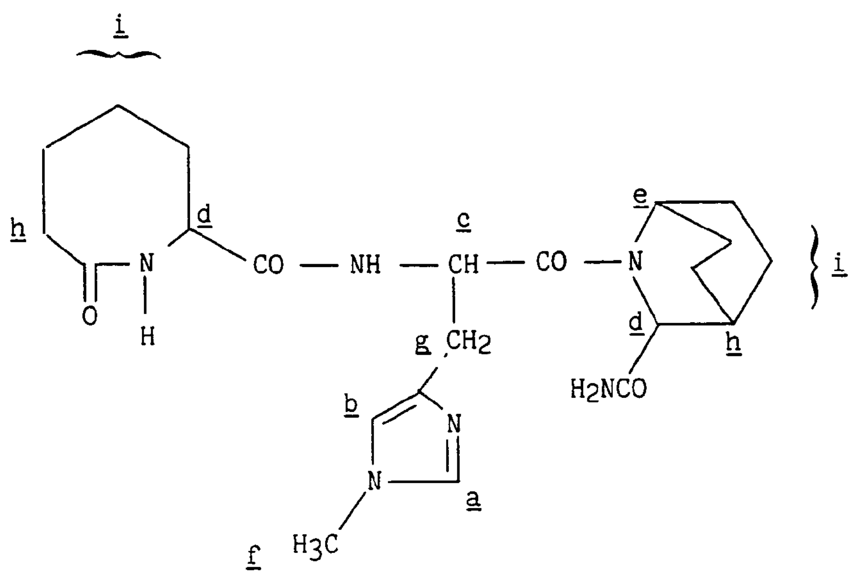

a δ = 7,5 ppm (1H, s)
b δ = 6,9 ppm (1H, s)
c δ = 4,85 ppm (1H, m)
d δ = 4,05 ppm (2H, m)
e δ = 3,8 ppm (1H, m)
f δ = 3,6 ppm (3H, s)
g δ entre 3,0 et 2,6 ppm (2H, m)
h δ entre 2,4 et 2,1 ppm (3H, m)
i δ entre 2,0 et 1,3 ppm (1H, m)

**EXEMPLE 48 : AZEP-(S)($N^{\tau}$-Me)His-(S)ABO-$NH_2$, "isomère B"**

L'isomère "B" est obtenu après élution de l'isomère "A" de l'exemple 47.

Résonance Magnétique Nucléaire du Proton (DMSO $D_6$) :

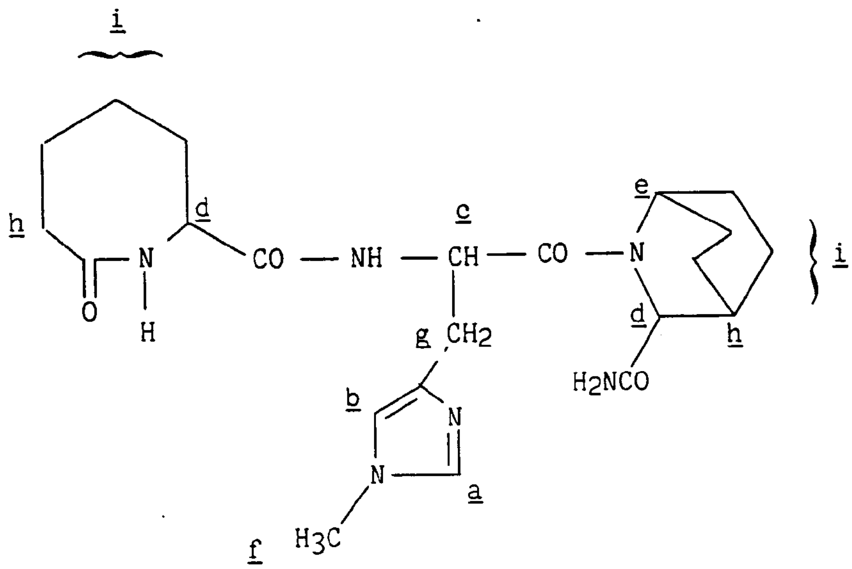

a δ = 7,5 ppm (1H, s)
b δ = 6,9 ppm (1H, s)
c δ = 4,85 ppm (1H, m)
d δ = 4,05 ppm (2H, m)
e δ = 3,8 ppm (1H, m)
f δ = 3,6 ppm (3H, s)
g δ entre 3,0 et 2,6 ppm (2H, m)
h δ entre 2,4 et 2,1 ppm (3H, m)
i δ entre 2,0 et 1,3 ppm (1H, m)

**EXEMPLE 49 : AZEP-(S)($N^{\tau}$-Me)His-(2S,3aS,7aS)PHI-$NH_2$, "isomère A"**

Le produit attendu est obtenu en procédant comme dans l'exemple 1 mais en remplaçant le (1S,3S,4R)ABH-$NH_2$ par le (2S,3aS,7aS)PHI-$NH_2$.

Les isomères "A" et "B" sont séparés par chromatographie liquide ( colonne de silice $C_{18}$, solvant d'élution : eau/acétonitrile/acide acétique : 97,5/2,5/0,1).

Microanalyse élémentaire :

| | C % | H % | N % |
|---|---|---|---|
| Calculé | 60,24 | 7,47 | 18,33 |
| Trouvé | 60,66 | 7,33 | 18,06 |

**EXEMPLE 50 : AZEP-(S)($N^{\tau}$-Me)His-(2S,3aS,7aS)PHI-$NH_2$, "isomère B"**

L'isomère "B" est obtenu après élution de l'isomère "A" de l'exemple 49.

Microanalyse élémentaire :

| | C % | H % | N % |
|---|---|---|---|
| Calculé | 60,24 | 7,47 | 18,33 |
| Trouvé | 60,34 | 7,17 | 17,91 |

**EXEMPLE 51 : AZEP-(S)($N^{\tau}$-Me)His-THIQ-$NH_2$**

**EXEMPLE 52 : AZEP-(S)($N^{\tau}$-Me)His-ThiaPro-$NH_2$**

**ETUDE PHARMACOLOGIQUE DES DERIVES DE L'INVENTION**

**EXEMPLE 53 : Déficit cholinergique par narcose barbiturique chez la Souris**

Chez la Souris, la narcose au pentobarbital (60 mg/kg IP) entraine dans l'hippocampe un déficit très marqué (-70 %) et reproductible de la capture de choline sodium-dépendante (HACU). La diminution de ce facteur limitant de la synthèse de l'acétylcholine témoigne de l'inhibition de la neurotransmission cholinergique, système étroitement impliqué dans les fonctions mnésiques.

Lorsqu'elle est administrée simultanément avec le pentobarbital, la TRH s'oppose à la baisse de l'HACU (10 mg/kg IP : -35 %), ce qui n'est plus le cas si elle est administrée 30 minutes avant le pentobarbital (10 mg/kg IP : -1,9 % ; 30 mg/kg IP : +4,8 %). A l'inverse, les composés de l'invention s'opposent très significativement à la baisse de l'HACU même s'ils sont administrés par voie IP 30 minutes avant le déclenchement de la narcose :

Composé de l'exemple 1 : (3 mg/kg) : -61,1 %
(1 mg/kg) : -25,5 %
Composé de l'exemple 5 : (0,3 mg/Kg) : -31,1 %
Composé de l'exemple 10 : (1 mg/Kg) : -50,8 %

**EXEMPLE 54 : Tremblements à l'oxotrémorine chez la Souris**

Administrée à la dose de 0,5 mg/kg par voie IP, l'oxotrémorine, agoniste muscarinique non sélectif $M_1$-$M_2$, entraîne des symptômes cholinergiques d'origine centrale, tels que les tremblements. Chez les animaux témoins, le maximum d'effet trémorigène est observé après 15 minutes et cet effet disparaît totalement en 45 à 60 minutes.

L'administration de TRH (10 mg/kg IP), 30 minutes avant celle d'oxotrémorine, potentialise les tremblements (+50 %) à leur acmée d'intensité (15 minutes) mais ne les prolonge que très peu (15 minutes : +20 %). La dose minima active est de 5 mg/kg.

Dans les mêmes conditions, les composés de l'invention exercent aussi le même effet potentialisateur mais à doses minima actives beaucoup plus faibles et cet effet persiste encore 60 minutes après l'injection d'oxotrémorine. Ainsi, les doses minima actives sont par exemple les suivantes :

Composé de l'exemple 1 : 0,3 mg/Kg
Composé de l'exemple 5 : 0,1 mg/Kg

Lorsque le délai entre les administrations du produit étudié et de l'oxotrémorine est allongé, la TRH ne potentialise plus les tremblements mesurés à l'acmée, si elle est administrée 60 minutes avant l'agoniste muscarinique, alors que les dérivés de l'invention demeurent actifs même s'ils sont administrés 150 minutes auparavant.

**EXEMPLE 55 : Inhibition du réflexe de retournement à la xylazine chez le Rat**

L'administration d'un $\alpha$2 agoniste central, la xylazine, chez le Rat entraîne la perte du réflexe de retournement des animaux. Cet effet est antagonisé par la yohimbine ($\alpha$2 antagoniste) et par les agents facilitant la libération de noradrénaline.

La TRH antagonise l'effet de la xylazine et la dose efficace 50 est proche de 10 mg/kg IP. Dans les mêmes conditions, les DE50 des composés des exemples 1 et 5 sont respectivement de 0,1 et 0,3 mg/kg.

Les dérivés de l'invention facilitent donc la neurotransmission noradrénergique lorsque celle-ci est inhibée préalablement.

**Composition pharmaceutique**

**EXEMPLE 56** :

Formule de préparation pour 1000 comprimés dosés à 10 mg du composé de l'exemple 1 :

| | |
|---|---|
| Composé de l'exemple 1 | 10 g |
| Hydroxy propyl cellulose | 2 g |
| Amidon de blé | 10 g |
| Lactose | 100 g |
| Stéarate de magnésium | 3 g |
| Talc | 3 g |

## Revendications

**Revendications pour les Etats contractants suivants : AT, BE, CH, DE, DK, FR, GB, IT, LI, LU, NL, SE**

**1.** Composés de formule générale (I) :

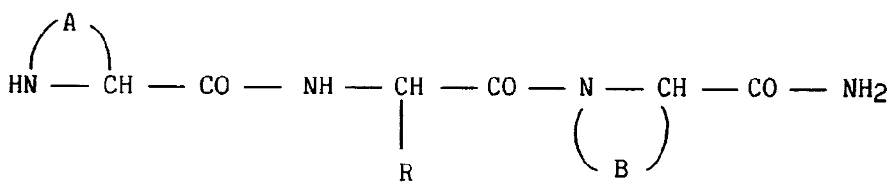

(I)

dans laquelle :

**A** représente avec les atomes d'azote et de carbone avec lesquels il est lié :
- un groupement 2-oxoperhydroazepin-7-yl
- un groupement 2-oxoperhydroazocin-8-yl
- un groupement 2-oxoperhydroazonin-9-yl
- un groupement 2-oxoperhydroazecin-10-yl
- un groupement 2-oxo-2,3,4,7-tétrahydrobenzo[e]azepin-7-yl
- un groupement 2-oxo-2,3,6,7-tétrahydrobenzo[d]azepin-7-yl
- un groupement 2-oxo-2,5,6,7-tétrahydrobenzo[c]azepin-7-yl

**B** représente avec les atomes d'azote et de carbone avec lesquels il est lié une structure polycyclique choisie parmi les structures suivantes :
- 2-azabicyclo [2.2.1] hept-2,3-ylène,
- 2-azabicyclo [2.2.2] oct-2,3-ylène éventuellement substitué en position 1 et 4 par un ou deux groupements alkyles ($C_1$-$C_4$) linéaires ou ramifiés,
- perhydroindol-1,2-ylène,
- perhydroisoindol-2,3-ylène,
- indol-1,2-ylène,
- isoindol-2,3-ylène,
- perhydroquinol-1,2-ylène,
- perhydroisoquinol-2,3-ylène,
- 1,2,3,4-tétrahydroquinol-1,2-ylène,
- 1,2,3,4-tétrahydroisoquinol-2,3-ylène,
- cyclopenta [b] pyrrolidin-1,2-ylène,
- pyrrolidin-1,2-ylène éventuellement substituée par un ou deux groupements alkyles ($C_1$-$C_4$) linéaires ou ramifiés,
- pipéridin-1,2-ylène,
- thiazolidin-3,4-ylène,

**R** représente :
- un atome d'hydrogène
- un groupement alkyle ($C_1$-$C_6$) linéaire ou ramifié éventuellement substitué par un groupement amino ou un groupement guanidino,

- un groupement (imidazol-4-yl) méthyle éventuellement substitué sur l'un des atomes d'azote par un radical alkyle ($C_1$-$C_4$) linéaire ou ramifié,
- un groupement (pyrazol-3-yl) méthyle,
- un groupement (pyridin-2-yl) méthyle éventuellement substitué par un groupement amino,

leurs énantiomères, diastéréoisomères et épimères, ainsi que leurs sels d'addition à un acide pharmaceutiquement acceptable.

**2.** Composés selon la revendication 1 tels que A forme avec les atomes d'azote et de carbone avec lesquels il est lié un cycle 2-oxoperhydroazepin-7-yl, leurs énantiomères, diastéréoisomères et épimères ainsi que leurs sels d'addition à un acide pharmaceutiquement acceptable.

**3.** Composés selon la revendication 1 tels que B forme avec les atomes d'azote et de carbone avec lesquels il est lié un cycle 2-azabicyclo[2.2.1]hept-2,3-ylène, leurs énantiomères, diastéréoisomères et épimères ainsi que leurs sels d'addition à un acide pharmaceutiquement acceptable.

**4.** Composé selon les revendications 1, 2 et 3 qui est le AZEP-($N^{\tau}$-Me)His-ABH-$NH_2$, ses énantiomères, diastéréoisomères et épimères ainsi que ses sels d'addition à un acide pharmaceutiquement acceptable, AZEP représentant le radical 7-carbonyl-perhydroazepin-2-one, ($N^{\tau}$-Me)His le radical 1-méthylhistidyle, et ABH le radical 2-aza-3-carbonylbicyclo[2.2.1]heptane.

**5.** Composé selon les revendications 1 et 2 qui est le AZEP-(S)His-(S)Pro-$NH_2$, ses énantiomères, diastéréoisomères et épimères ainsi que ses sels d'addition à un acide pharmaceutiquement acceptable, AZEP représentant le radical 7-carbonyl-perhydroazepin-2-one, His le radical histidyle et Pro le radical prolyle.

**6.** Procédé de préparation des dérivés de formule (I) caractérisé en ce que l'on protège la fonction amine d'un amino-acide de formule (II), dont on a éventuellement séparé les isomères par une technique classique de séparation :

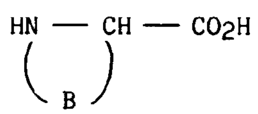

(II)

dans laquelle B a la même signification que dans la formule (I), par un radical protecteur (P) tel que le tert. butoxycarbonyle (tBOC) ou le benzyloxycarbonyle (Z) sous l'action d'un réactif approprié pour conduire à un dérivé de formule (III) :

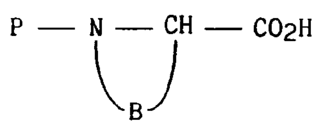

(III)

dans laquelle B et P ont la même signification que précédemment, sur lequel on fait réagir, à une température comprise entre -15 et 0°C, en présence de triéthylamine, le chloroformiate d'éthyle puis l'ammoniaque, pour conduire à un dérivé de formule (IV) :

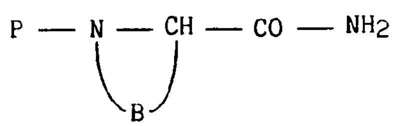

(IV)

dans laquelle B et P ont la même signification que précédemment,
que l'on déprotège par un procédé approprié comme par exemple l'action de l'acide chlorhydrique gazeux dans un solvant anhydre tel que le dioxane ou l'acétate d'éthyle dans le cas où P = tBOC ou par hydrogénation dans le cas où P=Z, pour conduire à un dérivé de formule (V) :

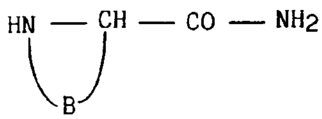 (V)

dans laquelle B a la même signification que dans la formule (I), dont on sépare, si on le souhaite, les isomères par une technique classique de séparation, qui est couplé avec un deuxième amino-acide protégé de formule (VI) :

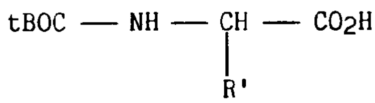 (VI)

dans laquelle R' représente un atome d'hydrogène, un groupement alkyle ($C_1$-$C_6$) linéaire ou ramifié éventuellement substitué par un groupement amino protégé par exemple par un radical benzyloxycarbonyle (Z) ou un groupement guanidino lui-même protégé par exemple par un radical nitro, un groupement (imidazol-4-yl) méthyle éventuellement substitué sur l'un des atomes d'azote par un radical alkyle ($C_1$-$C_4$) linéaire ou ramifié, un groupement (pyrazol-3-yl) méthyle, un groupement (pyridin-2-yl) méthyle éventuellement substitué par un groupement amino protégé par exemple par un radical benzyloxycarbonyle, pour conduire à un dérivé de formule (VII):

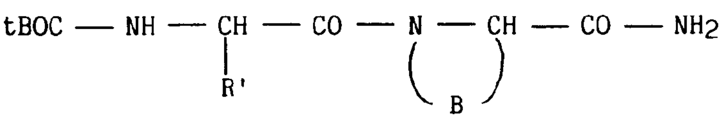 (VII)

dans laquelle R' et B ont la même signification que précédemment, dont on sépare, si on le souhaite, les diastéréoisomères ou énantiomères par une technique classique de séparation,
que l'on déprotège ensuite par action de l'acide chlorydrique gazeux dans un solvant anhydre comme par exemple le dioxanne ou l'acétate d'éthyle pour conduire à un dérivé de formule (VIII) :

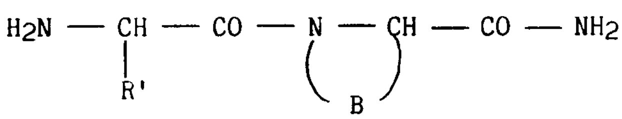 (VIII)

dans laquelle R' et B ont la même signification que précédemment, qui est couplé avec un troisième amino-acide, protégé de formule (IX) :

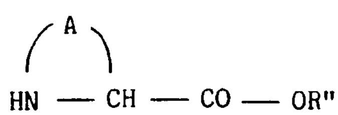 (IX)

dans laquelle R" est un radical succinimide pour conduire :

- ou bien :
  à un dérivé de formule (I) dans le cas où R' est différent d'un groupement alkyle ($C_1$-$C_6$) linéaire ou ramifié substitué par un groupement amino protégé, ou guanidino protégé ou différent d'un groupement (pyridin-2-yl) méthyle substitué par un groupement amino protégé,
  dont on sépare, si on le désire, les isomères selon une technique classique de séparation puis, si nécessaire, que l'on transforme en sel d'addition à un acide pharmaceutiquement acceptable,
- ou bien :
  à un dérivé de formule (X)

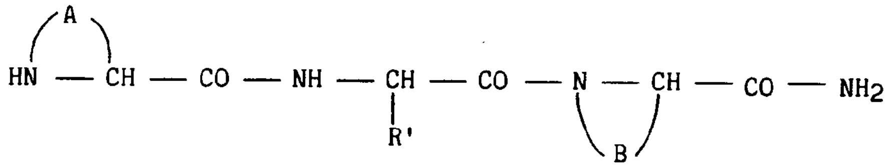

(X)

dans le cas où A et B ont la même signification que dans la formule (I) et R' est un groupement alkyle ($C_1$-$C_6$) linéaire ou ramifié substitué par un groupement amino protégé ou guanidino protégé ou R' est un groupement (pyridin-2-yl) méthyle substitué par un groupement amino protégé,

dont on sépare, si on le désire, les isomères selon une technique classique des séparation et que l'on déprotège par hydrogénation catalytique, par exemple, pour conduire à un dérivé de formule (I), dont on sépare éventuellement les isomères selon une technique classique de séparation puis, si nécessaire que l'on transforme en sel d'addition à un acide pharmaceutiquement acceptable.

**7.** Compositions pharmaceutiques contenant comme principe actif au moins un composé selon l'une quelconque des revendications 1 à 5 seul ou en combinaison avec un ou plusieurs excipients ou véhicules inertes, non toxiques, pharmaceutiquement acceptables.

**8.** Compositions pharmaceutiques selon la revendication 7 contenant au moins un principe actif selon l'une quelconque des revendications 1 à 5 utiles pour le traitement des désordres cognitifs et des troubles neurocomportementaux associés au vieillissement et aux maladies dégénératives du système nerveux, aigües ou chroniques comme la maladie d'Alzheimer, l'accident vasculaire cérébral, le traumatisme spinal ou la sclérose amyotrophique latérale.

**Revendications pour l'Etat contractant suivant : ES**

**1.** Procédé de préparation des composés de formule générale (I) :

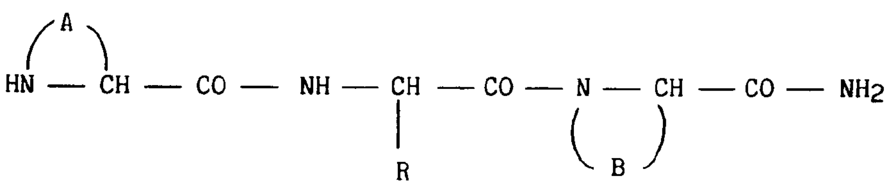

(I)

dans laquelle :

**A** représente avec les atomes d'azote et de carbone avec lesquels il est lié :
- un groupement 2-oxoperhydroazepin-7-yl
- un groupement 2-oxoperhydroazocin-8-yl
- un groupement 2-oxoperhydroazonin-9-yl
- un groupement 2-oxoperhydroazecin-10-yl
- un groupement 2-oxo-2,3,4,7-tétrahydrobenzo[e]azepin-7-yl
- un groupement 2-oxo-2,3,6,7-tétrahydrobenzo[d]azepin-7-yl
- un groupement 2-oxo-2,5,6,7-tétrahydrobenzo[c]azepin-7-yl

**B** représente avec les atomes d'azote et de carbone avec lesquels il est lié une structure polycyclique choisie parmi les structures suivantes :
- 2-azabicyclo [2.2.1] hept-2,3-ylène,
- 2-azabicyclo [2.2.2] oct-2,3-ylène éventuellement substitué en position 1 et 4 par un ou deux groupements alkyles ($C_1$-$C_4$) linéaires ou ramifiés,
- perhydroindol-1,2-ylène,
- perhydroisoindol-2,3-ylène,
- indol-1,2-ylène,
- isoindol-2,3-ylène,
- perhydroquinol-1,2-ylène,
- perhydroisoquinol-2,3-ylène,
- 1,2,3,4-tétrahydroquinol-1,2-ylène,
- 1,2,3,4-tétrahydroisoquinol-2,3-ylène,

- cyclopenta [b] pyrrolidin-1,2-ylène,
- pyrrolidin-1,2-ylène éventuellement substituée par un ou deux groupements alkyles ($C_1$-$C_4$) linéaires ou ramifiés,
- pipéridin-1,2-ylène,
- thiazolidin-3,4-ylène,

**R** représente :

- un atome d'hydrogène
- un groupement alkyle ($C_1$-$C_6$) linéaire ou ramifié éventuellement substitué par un groupement amino ou un groupement guanidino,
- un groupement (imidazol-4-yl) méthyle éventuellement substitué sur l'un des atomes d'azote par un radical alkyle ($C_1$-$C_4$) linéaire ou ramifié,
- un groupement (pyrazol-3-yl) méthyle,
- un groupement (pyridin-2-yl) méthyle éventuellement substitué par un groupement amino,

leurs énantiomères, diastéréoisomères et épimères, ainsi que leurs sels d'addition à un acide pharmaceutiquement acceptable,

caractérisé en ce que l'on protège la fonction amine d'un amino-acide de formule (II), dont on a éventuellement séparé les isomères par une technique classique de séparation :

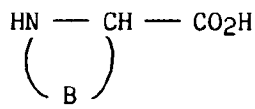

(II)

dans laquelle B a la même signification que dans la formule (I), par un radical protecteur (P) tel que le tert. butoxycarbonyle (tBOC) ou le benzyloxycarbonyle (Z) sous l'action d'un réactif approprié pour conduire à un dérivé de formule (III) :

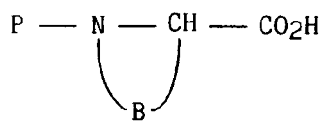

(III)

dans laquelle B et P ont la même signification que précédemment, sur lequel on fait réagir, à une température comprise entre -15 et 0°C, en présence de triéthylamine, le chloroformiate d'éthyle puis l'ammoniaque, pour conduire à un dérivé de formule (IV) :

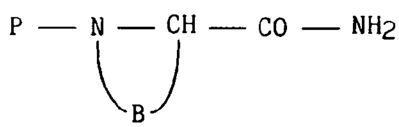

(IV)

dans laquelle B et P ont la même signification que précédemment,

que l'on déprotège par un procédé approprié comme par exemple l'action de l'acide chlorhydrique gazeux dans un solvant anhydre tel que le dioxane ou l'acétate d'éthyle dans le cas où P = tBOC ou par hydrogénation dans le cas où P=Z, pour conduire à un dérivé de formule (V) :

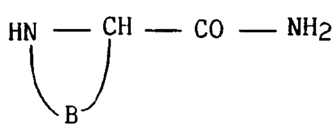

(V)

dans laquelle B a la même signification que dans la formule (I), dont on sépare, si on le souhaite, les isomères par une technique classique de séparation, qui est couplé avec un deuxième amino-acide protégé de formule (VI) :

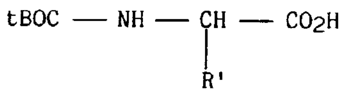

(VI)

dans laquelle R' représente un atome d'hydrogène, un groupement alkyle ($C_1$-$C_6$) linéaire ou ramifié éventuellement substitué par un groupement amino protégé par exemple par un radical benzyloxycarbonyle (Z) ou un groupement guanidino lui-même protégé par exemple par un radical nitro, un groupement (imidazol-4-yl) méthyle éventuellement substitué sur l'un des atomes d'azote par un radical alkyle ($C_1$-$C_4$) linéaire ou ramifié, un groupement (pyrazol-3-yl) méthyle, un groupement (pyridin-2-yl) méthyle éventuellement substitué par un groupement amino protégé par exemple par un radical benzyloxycarbonyle, pour conduire à un dérivé de formule (VII).

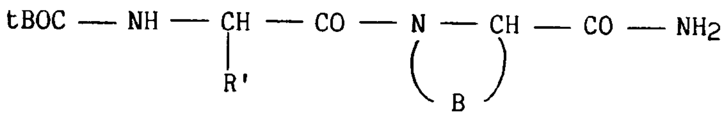

(VII)

dans laquelle R' et B ont la même signification que précédemment, dont on sépare, si on le souhaite, les diastéréoisomères ou énantiomères par une technique classique de séparation,
que l'on déprotège ensuite par action de l'acide chlorydrique gazeux dans un solvant anhydre comme par exemple le dioxanne ou l'acétate d'éthyle pour conduire à un dérivé de formule (VIII) :

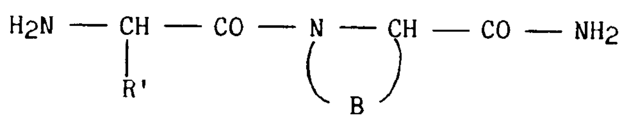

(VIII)

dans laquelle R' et B ont la même signification que précédemment, qui est couplé avec un troisième amino-acide, protégé de formule (IX) :

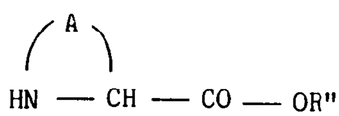

(IX)

dans laquelle R" est un radical succinimide pour conduire :

- ou bien :
  à un dérivé de formule (I) dans le cas où R' est différent d'un groupement alkyle ($C_1$-$C_6$) linéaire ou ramifié substitué par un groupement amino protégé, ou guanidino protégé ou différent d'un groupement (pyridin-2-yl) méthyle substitué par un groupement amino protégé,
  dont on sépare, si on le désire, les isomères selon une technique classique de séparation puis, si nécessaire, que l'on transforme en sel d'addition à un acide pharmaceutiquement acceptable,
- ou bien :
  à un dérivé de formule (X)

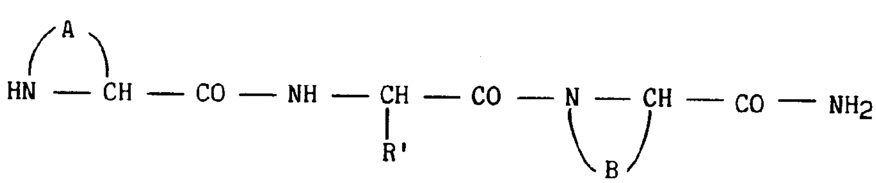

(X)

dans le cas où A et B ont la même signification que dans la formule (I) et R' est un groupement

alkyle ($C_1$-$C_6$) linéaire ou ramifié substitué par un groupement amino protégé ou guanidino protégé ou R' est un groupement (pyridin-2-yl) méthyle substitué par un groupement amino protégé,

dont on sépare, si on le désire, les isomères selon une technique classique de séparation et que l'on déprotège par hydrogénation catalytique, par exemple, pour conduire à un dérivé de formule (I), dont on sépare éventuellement les isomères selon une technique classique de séparation puis, si nécessaire que l'on transforme en sel d'addition à un acide pharmaceutiquement acceptable.

**2.** Procédé de préparation selon la revendication 1 des composés tels que A forme avec les atomes d'azote et de carbone avec lesquels il est lié un cycle 2-oxoperhydroazepin-7-yl, leurs énantiomères, diastéréoisomères et épimères ainsi que leurs sels d'addition à un acide pharmaceutiquement acceptable.

**3.** Procédé de préparation selon la revendication 1 des composés tels que B forme avec les atomes d'azote et de carbone avec lesquels il est lié un cycle 2-azabicyclo[2.2.1]heptan-2,3-ylène, leurs énantiomères, diastéréoisomères et épimères ainsi que leurs sels d'addition à un acide pharmaceutiquement acceptable.

**4.** Procédé de préparation selon la revendication 1 du composé qui est le AZEP-($N^{\tau}$-Me)His-ABH-$NH_2$, ses énantiomères, diastéréoisomères et épimères ainsi que ses sels d'addition à un acide pharmaceutiquement acceptable, AZEP représentant le radical 7-carbonyl-perhydroazepin-2-one, ($N^{\tau}$-Me)His le radical 1-méthylhistidyle, et ABH le radical 2-aza-3-carbonylbicyclo[2.2.1]heptane.

**5.** Procédé de préparation selon la revendication 1 du composé qui est le AZEP-(S)His-(S)Pro-$NH_2$, ses énantiomères, diastéréoisomères et épimères ainsi que ses sels d'addition à un acide pharmaceutiquement acceptable, AZEP représentant le radical 7-carbonyl-perhydroazepin-2-one, His le radical histidyle et Pro le radical prolyle.

**Revendications pour l'Etat contractant suivant : GR**

**1.** Procédé de préparation des composés de formule générale (I) :

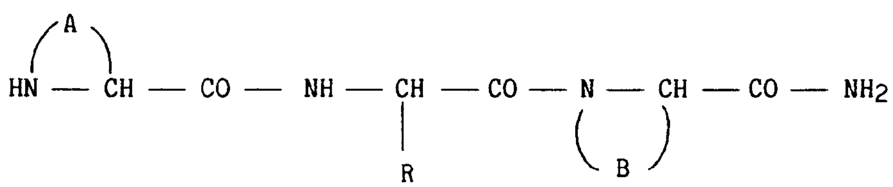

(I)

dans laquelle :

**A** représente avec les atomes d'azote et de carbone avec lesquels il est lié :
- un groupement 2-oxoperhydroazepin-7-yl
- un groupement 2-oxoperhydroazocin-8-yl
- un groupement 2-oxoperhydroazonin-9-yl
- un groupement 2-oxoperhydroazecin-10-yl
- un groupement 2-oxo-2,3,4,7-tétrahydrobenzo[e]azepin-7-yl
- un groupement 2-oxo-2,3,6,7-tétrahydrobenzo[d]azepin-7-yl
- un groupement 2-oxo-2,5,6,7-tétrahydiobenzo[c]azepin-7-yl

**B** représente avec les atomes d'azote et de carbone avec lesquels il est lié une structure polycyclique choisie parmi les structures suivantes :
- 2-azabicyclo [2.2.1] hept-2,3-ylène,
- 2-azabicyclo [2.2.2] oct-2,3-ylène éventuellement substitué en position 1 et 4 par un ou deux groupements alkyles ($C_1$-$C_4$) linéaires ou ramifiés,
- perhydroindol-1,2-ylène,
- perhydroisoindol-2,3-ylène,
- indol-1,2-ylène,
- isoindol-2,3-ylène,
- perhydroquinol-1,2-ylène,
- perhydroisoquinol-2,3-ylène,
- 1,2,3,4-tétrahydroquinol-1,2-ylène,
- 1,2,3,4-tétrahydroisoquinol-2,3-ylène,

- cyclopenta [b] pyrrolidin-1,2-ylène,
- pyrrolidin-1,2-ylène éventuellement substituée par un ou deux groupements alkyles ($C_1$-$C_4$) linéaires ou ramifiés,
- pipéridin-1,2-ylène,
- thiazolidin-3,4-ylène,

**R** représente :

- un atome d'hydrogène
- un groupement alkyle ($C_1$-$C_6$) linéaire ou ramifié éventuellement substitué par un groupement amino ou un groupement guanidino,
- un groupement (imidazol-4-yl) méthyle éventuellement substitué sur l'un des atomes d'azote par un radical alkyle ($C_1$-$C_4$) linéaire ou ramifié,
- un groupement (pyrazol-3-yl) méthyle,
- un groupement (pyridin-2-yl) méthyle éventuellement substitué par un groupement amino,

leurs énantiomères, diastéréoisomères et épimères, ainsi que leurs sels d'addition à un acide pharmaceutiquement acceptable,

caractérisé en ce que l'on protège la fonction amine d'un amino-acide de formule (II), dont on a éventuellement séparé les isomères par une technique classique de séparation :

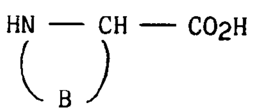

(II)

dans laquelle B a la même signification que dans la formule (I), par un radical protecteur (P) tel que le tert. butoxycarbonyle (tBOC) ou le benzyloxycarbonyle (Z) sous l'action d'un réactif approprié pour conduire à un dérivé de formule (III) :

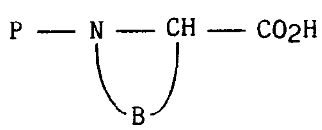

(III)

dans laquelle B et P ont la même signification que précédemment, sur lequel on fait réagir, à une température comprise entre -15 et 0°C, en présence de triéthylamine, le chloroformiate d'éthyle puis l'ammoniaque, pour conduire à un dérivé de formule (IV) :

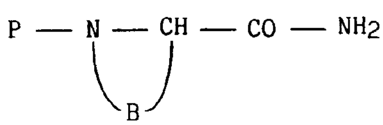

(IV)

dans laquelle B et P ont la même signification que précédemment,

que l'on déprotège par un procédé approprié comme par exemple l'action de l'acide chlorhydrique gazeux dans un solvant anhydre tel que le dioxane ou l'acétate d'éthyle dans le cas où P = tBOC ou par hydrogénation dans le cas où P=Z, pour conduire à un dérivé de formule (V) :

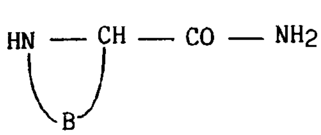

(V)

dans laquelle B a la même signification que dans la formule (I), dont on sépare, si on le souhaite, les isomères par une technique classique de séparation, qui est couplé avec un deuxième amino-acide protégé de formule (VI) :

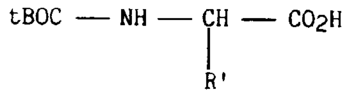

(VI)

dans laquelle R' représente un atome d'hydrogène, un groupement alkyle ($C_1$-$C_6$) linéaire ou ramifié éventuellement substitué par un groupement amino protégé par exemple par un radical benzyloxycarbonyle (Z) ou un groupement guanidino lui-même protégé par exemple par un radical nitro, un groupement (imidazol-4-yl) méthyle éventuellement substitué sur l'un des atomes d'azote par un radical alkyle ($C_1$-$C_4$) linéaire ou ramifié, un groupement (pyrazol-3-yl) méthyle, un groupement (pyridin-2-yl) méthyle éventuellement substitué par un groupement amino protégé par exemple par un radical benzyloxycarbonyle, pour conduire à un dérivé de formule (VII).

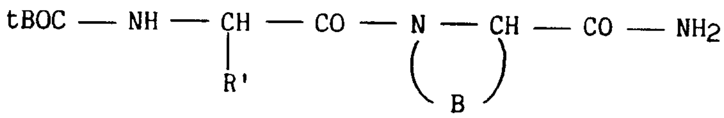

(VII)

dans laquelle R' et B ont la même signification que précédemment, dont on sépare, si on le souhaite, les diastéréoisomères ou énantiomères par une technique classique de séparation,
que l'on déprotège ensuite par action de l'acide chlorydrique gazeux dans un solvant anhydre comme par exemple le dioxanne ou l'acétate d'éthyle pour conduire à un dérivé de formule (VIII) :

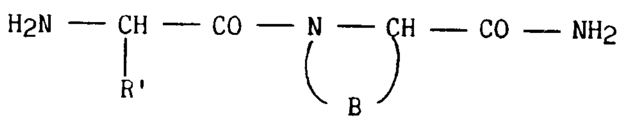

(VIII)

dans laquelle R' et B ont la même signification que précédemment, qui est couplé avec un troisième amino-acide, protégé de formule (IX) :

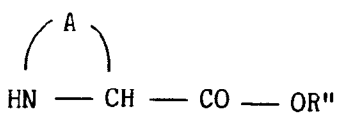

(IX)

dans laquelle R" est un radical succinimide pour conduire :

- ou bien :
  à un dérivé de formule (I) dans le cas où R' est différent d'un groupement alkyle ($C_1$-$C_6$) linéaire ou ramifié substitué par un groupement amino protégé, ou guanidino protégé ou différent d'un groupement (pyridin-2-yl) méthyle substitué par un groupement amino protégé,
  dont on sépare, si on le désire, les isomères selon une technique classique de séparation puis, si nécessaire, que l'on transforme en sel d'addition à un acide pharmaceutiquement acceptable,
- ou bien :
  à un dérivé de formule (X)

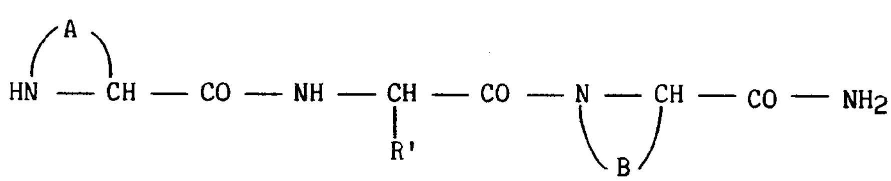

(X)

dans le cas où A et B ont la même signification que dans la formule (I) et R' est un groupement

alkyle ($C_1$-$C_6$) linéaire ou ramifié substitué par un groupement amino protégé ou guanidino protégé ou R' est un groupement (pyridin-2-yl) méthyle substitué par un groupement amino protégé,

dont on sépare, si on le désire, les isomères selon une technique classique de séparation et que l'on déprotège par hydrogénation catalytique, par exemple, pour conduire à un dérivé de formule (I), dont on sépare éventuellement les isomères selon une technique classique de séparation puis, si nécessaire que l'on transforme en sel d'addition à un acide pharmaceutiquement acceptable.

2. Procédé de préparation selon la revendication 1 des composés tels que A forme avec les atomes d'azote et de carbone avec lesquels il est lié un cycle 2-oxoperhydroazepin-7-yl, leurs énantiomères, diastéréoisomères et épimères ainsi que leurs sels d'addition à un acide pharmaceutiquement acceptable.

3. Procédé de préparation selon la revendication 1 des composés tels que B forme avec les atomes d'azote et de carbone avec lesquels il est lié un cycle 2-azabicyclo[2.2.1]heptan-2,3-ylène, leurs énantiomères, diastéréoisomères et épimères ainsi que leurs sels d'addition à un acide pharmaceutiquement acceptable.

4. Procédé de préparation selon la revendication 1 du composé qui est le AZEP-($N^{\tau}$-Me)His-ABH-$NH_2$, ses énantiomères, diastéréoisomères et épimères ainsi que ses sels d'addition à un acide pharmaceutiquement acceptable, AZEP représentant le radical 7-carbonyl-perhydroazepin-2-one, ($N^{\tau}$-Me)His le radical 1-méthylhistidyle, et ABH le radical 2-aza-3-carbonylbicyclo[2.2.1]heptane.

5. Procédé de préparation selon la revendication 1 du composé qui est le AZEP-(S)His-(S)Pro-$NH_2$, ses énantiomères, diastéréoisomères et épimères ainsi que ses sels d'addition à un acide pharmaceutiquement acceptable, AZEP représentant le radical 7-carbonyl-perhydroazepin-2-one, His le radical histidyle et Pro le radical prolyle.

## Claims

**Claims for the following Contracting States : AT, BE, CH, DE, DK, FR, GB, IT, LI, LU, NL, SE**

1. Compounds of the general formula (I):

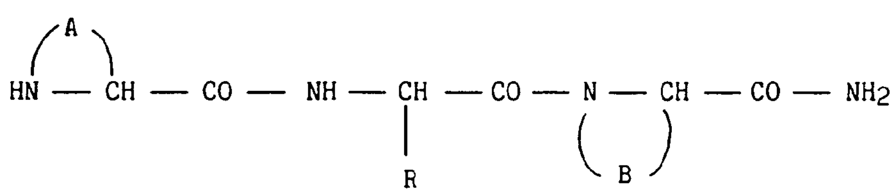

(I)

in which:

A represents, together with the nitrogen and carbon atoms to which it is attached:
- a 2-oxoperhydroazepin-7-yl grouping
- a 2-oxoperhydroazocin-8-yl grouping
- a 2-oxoperhydroazonin-9-yl grouping
- a 2-oxoperhydroazecin-10-yl grouping
- a 2-oxo-2,3,4,7-tetrahydrobenzo[e]azepin-7-yl grouping
- a 2-oxo-2,3,6,7-tetrahydrobenzo[d]azepin-7-yl grouping, or
- a 2-oxo-2,5,6,7-tetrahydrobenzo[c]azepin-7-yl grouping,

B represents, together with the nitrogen and carbon atoms to which it is attached, a polycyclic structure selected from the following structures:
- 2-azabicyclo[2.2.1]hept-2,3-ylene,
- 2-azabicyclo[2.2.2]oct-2,3-ylene optionally substituted in the 1- and 4-positions by one or two linear or branched ($C_1$-$C_4$)-alkyl groupings,
- perhydroindol-1,2-ylene,
- perhydroisoindol-2,3-ylene,
- indol-1,2-ylene,
- isoindol-2,3-ylene,

- perhydroquinol-1,2-ylene,
- perhydroisoquinol-2,3-ylene,
- 1,2,3,4-tetrahydroquinol-1,2-ylene,
- 1,2,3,4-tetrahydroisoquinol-2,3-ylene,
- cyclopenta[b]pyrrolidin-1,2-ylene,
- pyrrolidin-1,2-ylene optionally substituted by one or two linear or branched ($C_1$-$C_4$)-alkyl groupings,
- piperidin-1,2-ylene, or
- thiazolidin-3,4-ylene,

R represents:

- a hydrogen atom
- a linear or branched ($C_1$-$C_6$)-alkyl grouping optionally substituted by an amino grouping or by a guanidino grouping,
- an (imidazol-4-yl)methyl grouping optionally substituted at one of the nitrogen atoms by a linear or branched ($C_1$-$C_4$)-alkyl radical,
- a (pyrazol-3-yl)methyl grouping, or
- a (pyridin-2-yl)methyl grouping optionally substituted by an amino grouping,

their enantiomers, diastereoisomers and epimers, and also their addition salts with a pharmaceutically acceptable acid.

**2.** Compounds according to claim 1 in which A forms, together with the nitrogen and carbon atoms to which it is attached, a 2-oxoperhydroazepin-7-yl ring, their enantiomers, diastereoisomers and epimers, and also their addition salts with a pharmaceutically acceptable acid.

**3.** Compounds according to claim 1 in which B forms, together with the nitrogen and carbon atoms to which it is attached, a 2-azabicyclo[2.2.1]hept-2,3-ylene ring, their enantiomers, diastereoisomers and epimers, and also their addition salts with a pharmaceutically acceptable acid.

**4.** Compound according to claims 1, 2 and 3 that is AZEP-($N^{\tau}$-Me)His-ABH-$NH_2$, its enantiomers, diastereoisomers and epimers, and also its addition salts with a pharmaceutically acceptable acid, AZEP representing the radical 7-carbonylperhydroazepin-2-one, ($N^{\tau}$-Me)His the radical 1-methylhistidyl, and ABH the radical 2-aza-3-carbonylbicyclo[2.2.1]heptane.

**5.** Compound according to claims 1 and 2 that is AZEP-(S)His-(S)Pro-$NH_2$, its enantiomers, diastereoisomers and epimers, and also its addition salts with a pharmaceutically acceptable acid, AZEP representing the radical 7-carbonylperhydroazepin-2-one, His the radical histidyl and Pro the radical prolyl.

**6.** Process for the preparation of the compounds of the formula (I), characterised in that the amine function of an amino acid of the formula (II), of which the isomers have optionally been separated by a conventional separation technique:

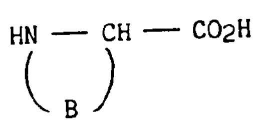

(II)

in which B is as defined in formula (I), is protected by a protecting radical (P), such as tert-butoxycarbonyl (tBOC) or benzyloxycarbonyl (Z), under the action of a suitable reagent to yield a compound of the formula (III):

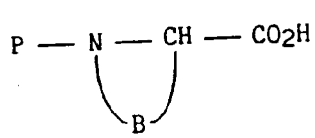

(III)

in which B and P are as defined above, which is reacted, in the presence of triethylamine, at a temperature of from -15 to 0°C, with ethyl chloroformate and then ammonia, to yield a compound of the formula (IV):

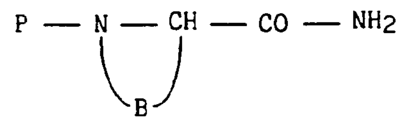

(IV)

in which B and P are as defined above,
which is deprotected by a suitable process, such as, for example, the action of gaseous hydrogen chloride in an anhydrous solvent, such as dioxane or ethyl acetate, in the case where P = tBOC, or by hydrogenation in the case where P = Z, to yield a compound of the formula (V):

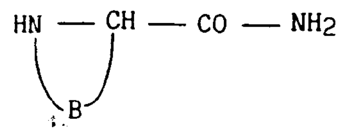

(V)

in which B is as defined in formula (I), of which, if desired, the isomers are separated by a conventional separation technique, and which is coupled with a second protected amino acid of the formula (VI):

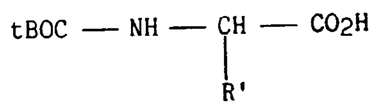

(VI)

in which R' represents a hydrogen atom, a linear or branched ($C_1$-$C_6$)-alkyl grouping that is optionally substituted by an amino grouping protected, for example, by a benzyloxycarbonyl radical (Z) or that is optionally substituted by a guanidino grouping itself protected, for example, by a nitro radical, an (imidazol-4-yl)methyl grouping optionally substituted at one of the nitrogen atoms by a linear or branched ($C_1$-$C_4$)-alkyl radical, a (pyrazol-3-yl)methyl grouping, or a (pyridin-2-yl)methyl grouping optionally substituted by an amino grouping that is protected, for example, by a benzyloxycarbonyl radical, to yield a compound of the formula (VII):

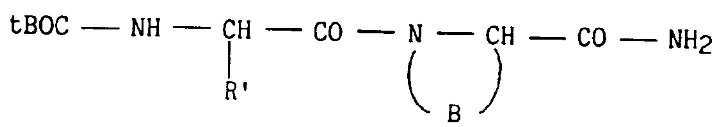

(VII)

in which R' and B are as defined above, of which, if desired, the diastereoisomers or enantiomers are separated by a conventional separation technique, and which is then deprotected by the action of gaseous hydrogen chloride in an anhydrous solvent, such as, for example, dioxane or ethyl acetate, to yield a compound of the formula (VIII):

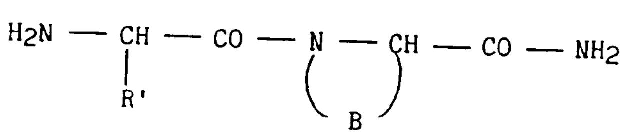

(VIII)

in which R' and B are as defined above, which is coupled with a third protected amino acid of the formula (IX):

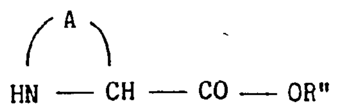

(IX)

in which R" is a succinimide radical, to yield:

- either:
  a compound of the formula (I) in the case where R' is other than a linear or branched ($C_1$-$C_6$)-alkyl grouping substituted by a protected amino grouping or by a protected guanidino grouping, and other than a (pyridin-2-yl)methyl grouping substituted by a protected amino grouping,
  of which, if desired, the isomers are separated according to a conventional separation technique, and which then, if necessary, is converted into an addition salt with a pharmaceutically acceptable acid,
- or:
  a compound of the formula (X):

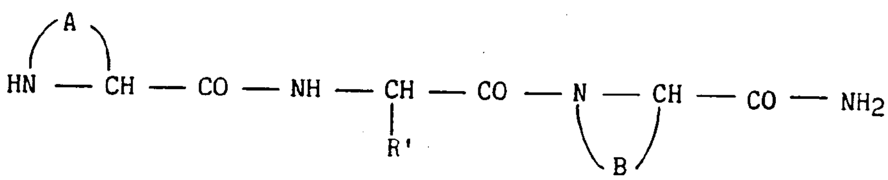

(X)

in the case where A and B are as defined in formula (I) and R' is a linear or branched ($C_1$-$C_6$)-alkyl grouping substituted by a protected amino grouping or by a protected guanidino grouping, or R' is a (pyridin-2-yl)methyl grouping substituted by a protected amino grouping,
of which, if desired, the isomers are separated according to a conventional separation technique and which is deprotected by catalytic hydrogenation, for example, to yield a compound of the formula (I) of which the isomers are optionally separated according to a conventional separation technique, and which then, if necessary, is converted into an addition salt with a pharmaceutically acceptable acid.

7. Pharmaceutical compositions containing as active ingredient at least one compound according to any one of claims 1 to 5, on its own or in combination with one or more pharmaceutically acceptable inert, non-toxic excipients or carriers.

8. Pharmaceutical compositions according to claim 7 containing at least one active ingredient according to any one of claims 1 to 5, which compositions can be used in the treatment of cognitive disorders and neuro-behavioural disorders associated with ageing and with acute or chronic degenerative diseases of the nervous system, such as Alzheimer's disease, cerebral vascular accident, spinal trauma or lateral amyotrophic sclerosis.

**Claims for the following Contracting State : ES**

1. Process for the preparation of compounds of the general formula (I):

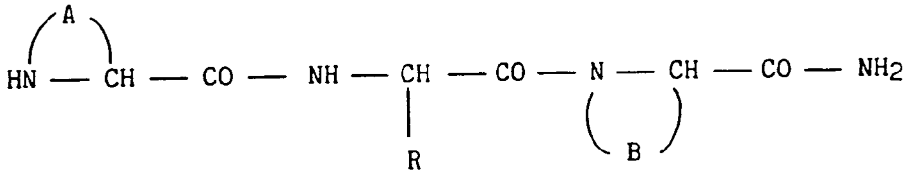

(I)

in which:

A represents, together with the nitrogen and carbon atoms to which it is attached:

- a 2-oxoperhydroazepin-7-yl grouping
- a 2-oxoperhydroazocin-8-yl grouping
- a 2-oxoperhydroazonin-9-yl grouping
- a 2-oxoperhydroazecin-10-yl grouping

- a 2-oxo-2,3,4,7-tetrahydrobenzo[e]azepin-7-yl grouping
- a 2-oxo-2,3,6,7-tetrahydrobenzo[d]azepin-7-yl grouping, or
- a 2-oxo-2,5,6,7-tetrahydrobenzo[c]azepin-7-yl grouping,

B represents, together with the nitrogen and carbon atoms to which it is attached, a polycyclic structure selected from the following structures:

- 2-azabicyclo[2.2.1]hept-2,3-ylene,
- 2-azabicyclo[2.2.2]oct-2,3-ylene optionally substituted in the 1- and 4-positions by one or two linear or branched ($C_1$-$C_4$)-alkyl groupings,
- perhydroindol-1,2-ylene,
- perhydroisoindol-2,3-ylene,
- indol-1,2-ylene,
- isoindol-2,3-ylene,
- perhydroquinol-1,2-ylene,
- perhydroisoquinol-2,3-ylene,
- 1,2,3,4-tetrahydroquinol-1,2-ylene,
- 1,2,3,4-tetrahydroisoquinol-2,3-ylene,
- cyclopenta[b]pyrrolidin-1,2-ylene,
- pyrrolidin-1,2-ylene optionally substituted by one or two linear or branched ($C_1$-$C_4$)-alkyl groupings,
- piperidin-1,2-ylene, or
- thiazolidin-3,4-ylene,

R represents:

- a hydrogen atom
- a linear or branched ($C_1$-$C_6$)-alkyl grouping optionally substituted by an amino grouping or by a guanidino grouping,
- an (imidazol-4-yl)methyl grouping optionally substituted at one of the nitrogen atoms by a linear or branched ($C_1$-$C_4$)-alkyl radical,
- a (pyrazol-3-yl)methyl grouping, or
- a (pyridin-2-yl)methyl grouping optionally substituted by an amino grouping,

their enantiomers, diastereoisomers and epimers, and also their addition salts with a pharmaceutically acceptable acid,

characterised in that the amine function of an amino acid of the formula (II), of which the isomers have optionally been separated by a conventional separation technique:

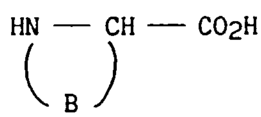

(II)

in which B is as defined in formula (I), is protected by a protecting radical (P), such as tert-butoxycarbonyl (tBOC) or benzyloxycarbonyl (Z), under the action of a suitable reagent to yield a compound of the formula (III):

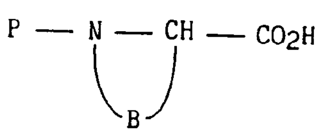

(III)

in which B and P are as defined above, which is reacted, in the presence of triethylamine, at a temperature of from -15 to 0°C, with ethyl chloroformate and then ammonia, to yield a compound of the formula (IV):

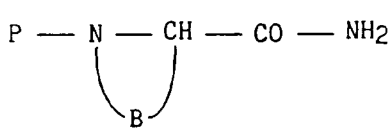

(IV)

in which B and P are as defined above,
which is deprotected by a suitable process, such as, for example, the action of gaseous hydrogen chloride in an anhydrous solvent, such as dioxane or ethyl acetate, in the case where P = tBOC, or by hydrogenation in the case where P = Z, to yield a compound of the formula (V):

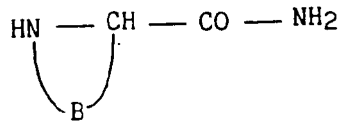

(V)

in which B is as defined in formula (I), of which, if desired, the isomers are separated by a conventional separation technique, and which is coupled with a second protected amino acid of the formula (VI):

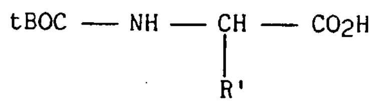

(VI)

in which R' represents a hydrogen atom, a linear or branched ($C_1$-$C_6$)-alkyl grouping that is optionally substituted by an amino grouping protected, for example, by a benzyloxycarbonyl radical (Z) or that is optionally substituted by a guanidino grouping itself protected, for example, by a nitro radical, an (imidazol-4-yl)methyl grouping optionally substituted at one of the nitrogen atoms by a linear or branched ($C_1$-$C_4$)-alkyl radical, a (pyrazol-3-yl)methyl grouping, or a (pyridin-2-yl)methyl grouping optionally substituted by an amino grouping that is protected, for example, by a benzyloxycarbonyl radical, to yield a compound of the formula (VII):

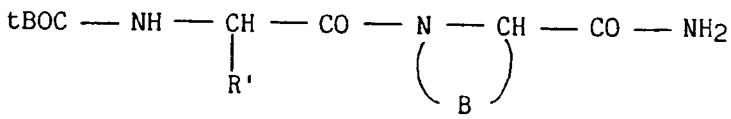

(VII)

in which R' and B are as defined above, of which, if desired, the diastereoisomers or enantiomers are separated by a conventional separation technique, and which is then deprotected by the action of gaseous hydrogen chloride in an anhydrous solvent, such as, for example, dioxane or ethyl acetate, to yield a compound of the formula (VIII):

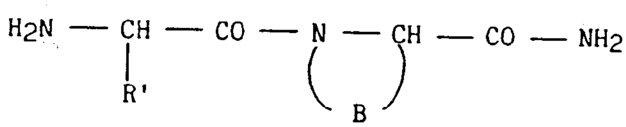

(VIII)

in which R' and B are as defined above, which is coupled with a third protected amino acid of the formula (IX):

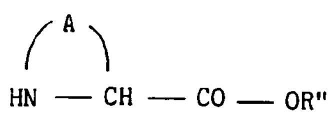

(IX)

in which R" is a succinimide radical, to yield:

- either:
  a compound of the formula (I) in the case where R' is other than a linear or branched ($C_1$-$C_6$)-alkyl grouping substituted by a protected amino grouping or by a protected guanidino grouping, and other

than a (pyridin-2-yl)methyl grouping substituted by a protected amino grouping,
of which, if desired, the isomers are separated according to a conventional separation technique, and which then, if necessary, is converted into an addition salt with a pharmaceutically acceptable acid,

- or:
  a compound of the formula (X):

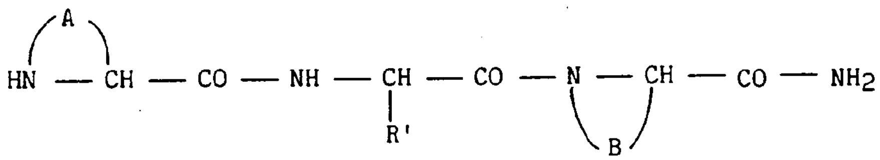

(X)

in the case where A and B are as defined in formula (I) and R' is a linear or branched ($C_1$-$C_6$)-alkyl grouping substituted by a protected amino grouping or by a protected guanidino grouping, or R' is a (pyridin-2-yl)methyl grouping substituted by a protected amino grouping,
of which, if desired, the isomers are separated according to a conventional separation technique and which is deprotected by catalytic hydrogenation, for example, to yield a compound of the formula (I) of which the isomers are optionally separated according to a conventional separation technique, and which then, if necessary, is converted into an addition salt with a pharmaceutically acceptable acid.

**2.** Process for the preparation according to claim 1 of compounds in which A forms, together with the nitrogen and carbon atoms to which it is attached, a 2-oxoperhydroazepin-7-yl ring, their enantiomers, diastereoisomers and epimers, and also their addition salts with a pharmaceutically acceptable acid.

**3.** Process for the preparation according to claim 1 of compounds in which B forms, together with the nitrogen and carbon atoms to which it is attached, a 2-azabicyclo-[2.2.1]hept-2,3-ylene ring, their enantiomers, diastereoisomers and epimers, and also their addition salts with a pharmaceutically acceptable acid.

**4.** Process for the preparation according to claim 1 of the compound that is AZEP-($N^\tau$-Me)His-ABH-$NH_2$, its enantiomers, diastereoisomers and epimers, and also its addition salts with a pharmaceutically acceptable acid, AZEP representing the radical 7-carbonylperhydroazepin-2-one, ($N^\tau$-Me)His the radical 1-methylhistidyl, and ABH the radical 2-aza-3-carbonylbicyclo[2.2.1]heptane.

**5.** Process for the preparation according to claim 1 of the compound that is AZEP-(S)His-(S)Pro-$NH_2$, its enantiomers, diastereoisomers and epimers, and also its addition salts with a pharmaceutically acceptable acid, AZEP representing the radical 7-carbonylperhydroazepin-2-one, His the radical histidyl and Pro the radical prolyl.

**Claims for the following Contracting State : GR**

**1.** Process for the preparation of compounds of the general formula (I):

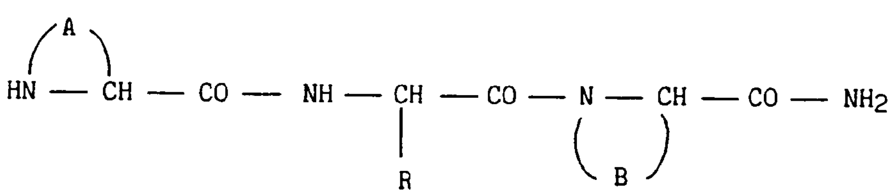

(I)

in which:
A represents, together with the nitrogen and carbon atoms to which it is attached:

- a 2-oxoperhydroazepin-7-yl grouping
- a 2-oxoperhydroazocin-8-yl grouping
- a 2-oxoperhydroazonin-9-yl grouping
- a 2-oxoperhydroazecin-10-yl grouping
- a 2-oxo-2,3,4,7-tetrahydrobenzo[e]azepin-7-yl grouping
- a 2-oxo-2,3,6,7-tetrahydrobenzo[d]azepin-7-yl grouping, or

- a 2-oxo-2,5,6,7-tetrahydrobenzo[c]azepin-7-yl grouping,

B represents, together with the nitrogen and carbon atoms to which it is attached, a polycyclic structure selected from the following structures:

- 2-azabicyclo[2.2.1]hept-2,3-ylene,
- 2-azabicyclo[2.2.2]oct-2,3-ylene optionally substituted in the 1- and 4-positions by one or two linear or branched ($C_1$-$C_4$)-alkyl groupings,
- perhydroindol-1,2-ylene,
- perhydroisoindol-2,3-ylene,
- indol-1,2-ylene,
- isoindol-2,3-ylene,
- perhydroquinol-1,2-ylene,
- perhydroisoquinol-2,3-ylene,
- 1,2,3,4-tetrahydroquinol-1,2-ylene,
- 1,2,3,4-tetrahydroisoquinol-2,3-ylene,
- cyclopenta[b]pyrrolidin-1,2-ylene,
- pyrrolidin-1,2-ylene optionally substituted by one or two linear or branched ($C_1$-$C_4$)-alkyl groupings,
- piperidin-1,2-ylene, or
- thiazolidin-3,4-ylene,

R represents:

- a hydrogen atom
- a linear or branched ($C_1$-$C_6$)-alkyl grouping optionally substituted by an amino grouping or by a guanidino grouping,
- an (imidazol-4-yl)methyl grouping optionally substituted at one of the nitrogen atoms by a linear or branched ($C_1$-$C_4$)-alkyl radical,
- a (pyrazol-3-yl)methyl grouping, or
- a (pyridin-2-yl)methyl grouping optionally substituted by an amino grouping,

their enantiomers, diastereoisomers and epimers, and also their addition salts with a pharmaceutically acceptable acid,

characterised in that the amine function of an amino acid of the formula (II), of which the isomers have optionally been separated by a conventional separation technique:

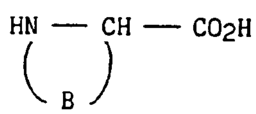

(II)

in which B is as defined in formula (I), is protected by a protecting radical (P), such as tert-butoxycarbonyl (tBOC) or benzyloxycarbonyl (Z), under the action of a suitable reagent to yield a compound of the formula (III):

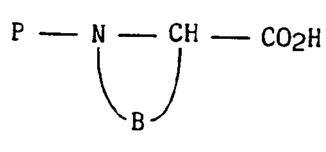

(III)

in which B and P are as defined above, which is reacted, in the presence of triethylamine, at a temperature of from -15 to 0°C, with ethyl chloroformate and then ammonia, to yield a compound of the formula (IV):

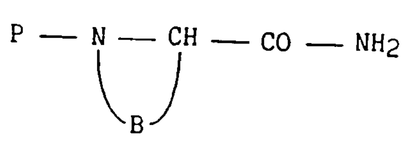

(IV)

in which B and P are as defined above,

which is deprotected by a suitable process, such as, for example, the action of gaseous hydrogen chloride

in an anhydrous solvent, such as dioxane or ethyl acetate, in the case where P = tBOC, or by hydrogenation in the case where P = Z, to yield a compound of the formula (V):

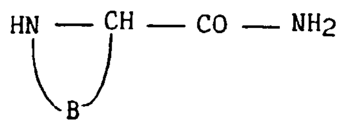

(V)

in which B is as defined in formula (I), of which, if desired, the isomers are separated by a conventional separation technique, and which is coupled with a second protected amino acid of the formula (VI):

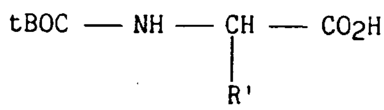

(VI)

in which R' represents a hydrogen atom, a linear or branched ($C_1$-$C_6$)-alkyl grouping that is optionally substituted by an amino grouping protected, for example, by a benzyloxycarbonyl radical (Z) or that is optionally substituted by a guanidino grouping itself protected, for example, by a nitro radical, an (imidazol-4-yl)methyl grouping optionally substituted at one of the nitrogen atoms by a linear or branched ($C_1$-$C_4$)-alkyl radical, a (pyrazol-3-yl)methyl grouping, or a (pyridin-2-yl)methyl grouping optionally substituted by an amino grouping that is protected, for example, by a benzyloxycarbonyl radical, to yield a compound of the formula (VII):

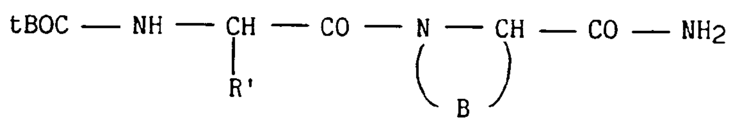

(VII)

in which R' and B are as defined above, of which, if desired, the diastereoisomers or enantiomers are separated by a conventional separation technique, and which is then deprotected by the action of gaseous hydrogen chloride in an anhydrous solvent, such as, for example, dioxane or ethyl acetate, to yield a compound of the formula (VIII):

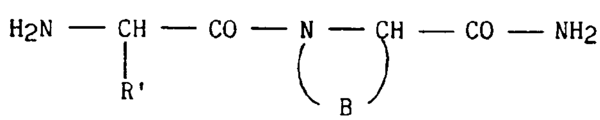

(VIII)

in which R' and B are as defined above, which is coupled with a third protected amino acid of the formula (IX):

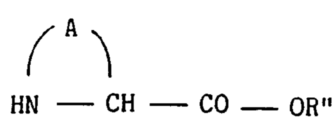

(IX)

in which R" is a succinimide radical, to yield:

- either:
  a compound of the formula (I) in the case where R' is other than a linear or branched ($C_1$-$C_6$)-alkyl grouping substituted by a protected amino grouping or by a protected guanidino grouping, and other than a (pyridin-2-yl)methyl grouping substituted by a protected amino grouping,
  of which, if desired, the isomers are separated according to a conventional separation technique, and which then, if necessary, is converted into an addition salt with a pharmaceutically acceptable acid,

- or:
  a compound of the formula (X):

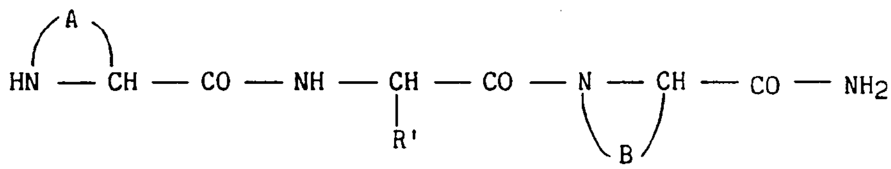

(X)

in the case where A and B are as defined in formula (I) and R' is a linear or branched ($C_1$-$C_6$)-alkyl grouping substituted by a protected amino grouping or by a protected guanidino grouping, or R' is a (pyridin-2-yl)methyl grouping substituted by a protected amino grouping,
of which, if desired, the isomers are separated according to a conventional separation technique and which is deprotected by catalytic hydrogenation, for example, to yield a compound of the formula (I) of which the isomers are optionally separated according to a conventional separation technique, and which then, if necessary, is converted into an addition salt with a pharmaceutically acceptable acid.

2. Process for the preparation according to claim 1 of compounds in which A forms, together with the nitrogen and carbon atoms to which it is attached, a 2-oxoperhydroazepin-7-yl ring, their enantiomers, diastereoisomers and epimers, and also their addition salts with a pharmaceutically acceptable acid.

3. Process for the preparation according to claim 1 of compounds in which B forms, together with the nitrogen and carbon atoms to which it is attached, a 2-azabicyclo-[2.2.1]hept-2,3-ylene ring, their enantiomers, diastereoisomers and epimers, and also their addition salts with a pharmaceutically acceptable acid.

4. Process for the preparation according to claim 1 of the compound that is AZEP-($N^{\tau}$-Me)His-ABH-$NH_2$, its enantiomers, diastereoisomers and epimers, and also its addition salts with a pharmaceutically acceptable acid, AZEP representing the radical 7-carbonylperhydroazepin-2-one, ($N^{\tau}$-Me)His the radical 1-methylhistidyl, and ABH the radical 2-aza-3-carbonylbicyclo[2.2.1]heptane.

5. Process for the preparation according to claim 1 of the compound that is AZEP-(S)His-(S)Pro-$NH_2$, its enantiomers, diastereoisomers and epimers, and also its addition salts with a pharmaceutically acceptable acid, AZEP representing the radical 7-carbonylperhydroazepin-2-one, His the radical histidyl and Pro the radical prolyl.

## Patentansprüche

### Patentansprüche für folgende Vertragsstaaten : AT, BE, CH, DE, DK, FR, GB, IT, LI, LU, NL, SE

1. Verbindungen der allgemeinen Formel (I):

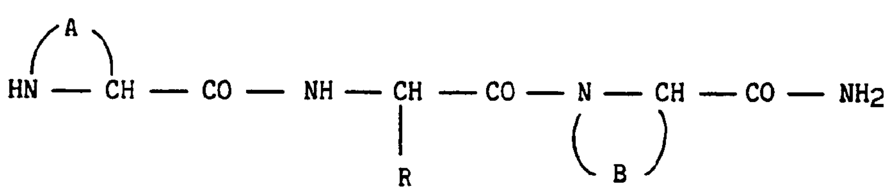

(I)

in der:
A zusammen mit dem Stickstoff- und dem Kohlenstoffatom, an die es gebunden ist:
- eine 2-Oxoperhydroazepin-7-yl-gruppe,
- eine 2-Oxoperhydroazocin-8-yl-gruppe,
- eine 2-Oxoperhydroazonin-9-yl-gruppe,
- eine 2-Oxoperhydroazecin-10-yl-gruppe,
- eine 2-Oxo-2,3,4,7-tetrahydrobenzo[e]azepin-7-yl-gruppe,

- eine 2-Oxo-2,3,6, 7-tetrahydrobenzo[d]azepin-7-yl-gruppe,
- eine 2-Oxo-2,5,6,7-tetrahydrobenzo[c]azepin-7-yl-gruppe bedeutet;

B zusammen mit dem Stickstoff- und dem Kohlenstoffatom, an die es gebunden ist, eine polycyclische Struktur bedeutet, die aus den folgenden Strukturen ausgewählt ist:

- 2-Azabicyclo[2.2.1]hept-2,3-ylen,
- 2-Azabicyclo[2.2.2]oct-2,3-ylen, das gegebenenfalls in der 1- und der 4-Stellung durch eine oder zwei geradkettige oder verzweigte $C_1$-$C_4$-Gruppen substituiert ist,
- Perhydroindol-1,2-ylen,
- Perhydroisoindol-2,3-ylen,
- Indol-1,2-ylen,
- Isoindol-2,3-ylen,
- Perhydrochinol-1,2-ylen,
- Perhydroisochinol-2,3-ylen,
- 1,2,3,4-Tetrahydrochinol-1,2-ylen,
- 1,2,3,4-Tetrahydroisochinol-2,3-ylen,
- Cyclopenta[b]pyrrolidin-1,2-ylen,
- Pyrrolidin-1,2-ylen, das gegebenenfalls durch eine oder zwei geradkettige oder verzweigte $C_1$-$C_4$-Alkylgruppen substituiert ist,
- Piperidin-1,2-ylen und
- Thiazolidin-3,4-ylen;

R - ein Wasserstoffatom,

- eine geradkettige oder verzweigte $C_1$-$C_6$-Alkylgruppe, die gegebenenfalls durch eine Aminogruppe oder eine Guanidinogruppe substituiert ist,
- eine (Imidazol-4-yl)-methylgruppe, die gegebenenfalls an einem der Stickstoffatome durch eine geradkettige oder verzweigte $C_1$-$C_4$-Alkylgruppe substituiert ist,
- eine (Pyrazol-3-yl)-methylgruppe oder
- eine (Pyridin-2-yl)-methylgruppe, die gegebenenfalls durch eine Aminogruppe substituiert ist, bedeutet,

sowie deren Enantiomere, Diastereoisomere und Epimere sowie deren Additionssalze mit einer pharmazeutisch annehmbaren Säure.

2. Verbindungen nach Anspruch 1, worin A zusammen mit dem Stickstoffund dem Kohlenstoffatom, an die es gebunden ist, einen 2-Oxoperhydroazepin-7-yl-Zyklus bedeutet, sowie deren Enantiomere, Diastereoisomere und Epimere sowie deren Additionssalze mit einer pharmazeutisch annehmbaren Säure.

3. Verbindungen nach Anspruch 1, in denen B zusammen mit dem Stickstoff- und dem Kohlenstoffatom, an die es gebunden ist, einen 2-Azabicyclo[2.2.1]hept-2,3-ylen-Zyklus bildet, sowie deren Enantiomere, Diastereoisomere und Epimere sowie deren Additionssalze mit einer pharmazeutisch annehmbaren Säure.

4. Verbindung nach den Ansprüchen 1, 2 und 3, nämlich AZEP-($N^{\tau}$-Me)-His-ABH-$NH_2$, dessen Enantiomere, Diastereoisomere und Epimere sowie dessen Additionssalze mit einer pharmazeutisch annehmbaren Säure, worin AZEP für den 7-Carbonyl-perhydroazepin-2-on-Rest, ($N^{\tau}$-Me)-His für den 1-Methylhistidylrest und ABH für den 2-Aza-3-carbonylbicyclo[2.2.1]heptan-Rest stehen.

5. Verbindung nach den Ansprüchen 1 und 2, nämlich AZEP-(S)-His-(S)-Pro-$NH_2$, dessen Enantiomere, Diastereoisomere und Epimere sowie dessen Additionssalze mit einer pharmazeutisch annehmbaren Säure, worin AZEP für den 7-Carbonyl-perhydroazepin-2-on-Rest, His für den Histidylrest und Pro für den Prolylrest stehen.

6. Verfahren zur Herstellung der Derivate der Formel (I), **dadurch gekennzeichnet**, daß man die Aminogruppe einer Aminosäure der Formel (II), die man gegebenenfalls mit Hilfe klassischer Trennmethoden in die Isomeren getrennt hat:

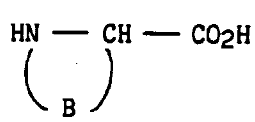

(II)

in der B die für die Formel (I) angegebenen Bedeutungen besitzt, mit einer Schutzgruppe (P), wie der tert.-Butoxycarbonylgruppe (tBOC) oder der Benzyloxycarbonylgruppe (Z), unter Einwirkung eines geeigneten Reagens schützt zur Bildung eines Derivats der Formel (III):

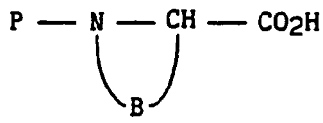

(III)

in der B und P die oben angegebenen Bedeutungen besitzen, welches man bei einer Temperatur zwischen -15 und 0°C in Gegenwart von Triethylamin mit Chlorameisensäureethylester und dann mit Ammoniak umsetzt zur Bildung eines Derivats der Formel (IV):

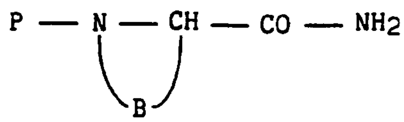

(IV)

in der B und P die oben angegebenen Bedeutungen besitzen,
von dem man die Schutzgruppe mit Hilfe eines geeigneten Verfahrens, beispielsweise durch Einwirkung von gasförmiger Chlorwasserstoffsäure in einem wasserfreien Lösungsmittel, wie Dioxan oder Ethylacetat, in dem Fall, da P = tBOC ist, oder durch Hydrieren dann, wenn P = Z ist, abspaltet zur Bildung eines Derivats der Formel (V):

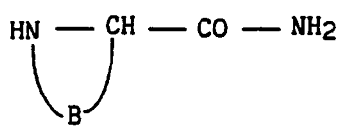

(V)

in der B die bezüglich der Formel (I) angegebenen Bedeutungen besitzt, welches man gewünschtenfalls mit Hilfe klassischer Trennungsmethoden in die Isomeren trennt, und welches man mit einer zweiten geschützten Aminosäure der Formel (VI):

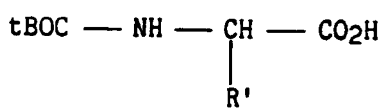

(VI)

in der R' ein Wasserstoffatom, eine geradkettige oder verzeigte $C_1$-$C_6$-Alkylgruppe, die gegebenenfalls durch eine Aminogruppe, die beispielsweise durch eine Benzyloxycarbonylgruppe (Z) geschützt ist, oder eine Guanidinogruppe, die ihrerseits beispielsweise durch eine Nitrogruppe geschützt ist, substituiert ist, eine (Imidazol-4-yl)-methylgruppe, die gegebenenfalls an einem der Stickstoffatome durch eine geradkettige oder verzweigte $C_1$-$C_4$-Alkylgruppe substituiert ist, eine (Pyrazol-3-yl)-methylgruppe, eine (Pyridin-2-yl)-methylgruppe, die gegebenenfalls durch eine Aminogruppe, die beispielsweise durch eine Benzyloxycarbonylgruppe geschützt ist, substituiert ist, bedeutet, kuppelt unter Bildung eines Derivats der Formel (VII):

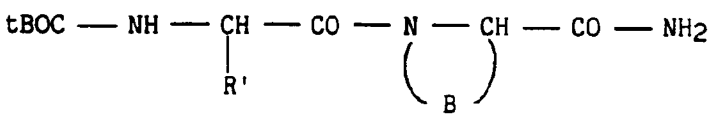

(VII)

in der R' und B die oben angegebenen Bedeutungen besitzen, das man gewünschtenfalls mit Hilfe einer klassischen Trennungsmethode in die Diastereoisomeren oder Enantiomeren auftrennt, von welchen man anschließend durch Einwirkung von gasförmiger Chlorwasserstoffsäure in einem wasserfreien Lösungsmittel, wie beispielsweise Dioxan oder Ethylacetat, die Schutzgruppe abspaltet zur Bildung eines Derivats der Formel (VIII):

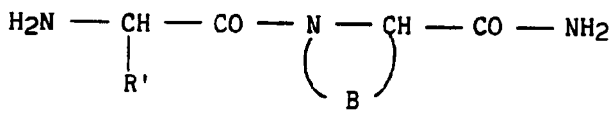

(VIII)

in der R' und B die oben angegebenen Bedeutungen besitzen, welches man mit einer dritten geschützten Aminosäure der Formel (IX):

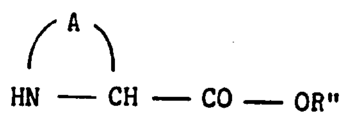

(IX)

in der R" einen Succinimidrest bedeutet, kuppelt, so daß man:

- entweder:
  ein Derivat der Formel (I) erhält, dann, wenn R' von einer geradkettigen oder verzweigten, durch eine geschützte Aminogruppe oder eine geschützte Guanidinogruppe substituierte $C_1$-$C_6$-Alkylgruppe oder von einer durch eine geschützte Aminogruppe substituierte (Pyridin-2-yl)-methylgruppe verschieden ist,
  welches man gewünschtenfalls mit Hilfe einer klassischen Trennungsmethode in die Isomeren auftrennt und welches man erforderlichenfalls in ein Salz mit einer pharmazeutisch annehmbaren Säure überführt,
- oder:
  ein Derivat der Formel (X)

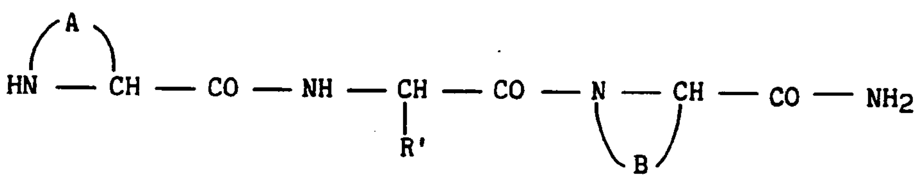

(X)

erhält, dann, wenn A und B die bezüglich der Formel (I) angegebenen Bedeutungen besitzen und R' eine geradkettige oder verzweigte, durch eine geschützte Aminogruppe oder eine geschützte Guanidinogruppe substituierte $C_1$-$C_6$-Alkylgruppe oder R' eine durch eine geschützte Aminogruppe substituierte (Pyridin-2-yl)-methylgruppe bedeutet,

welches man gewünschtenfalls mit Hilfe einer klassischen Trennungsmethode in die Isomeren aufspaltet und von dem man beispielsweise durch katalytische Hydrierung die Schutzgruppe abspaltet, so daß man ein Derivat der Formel (I) erhält, das man gegebenenfalls mit Hilfe einer klassischen Trennungsmethode in die Isomeren aufspaltet und das man erforderlichenfalls in ein Additionssalz mit einer pharmazeutisch annehmbaren Säure überführt.

7. Pharmazeutische Zubereitungen, enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien oder Hilfsstoffen.

8. Pharmazeutische Zubereitungen nach Anspruch 7, enthaltend mindestens einen Wirkstoff nach einem der Ansprüche 1 bis 5 zur Behandlung von Erkennungsstörungen und Störungen des Nervenverhaltens, die mit dem Altern verknüpft sind, und degenerativen akuten oder chronischen Erkrankungen des Zentralnervensystems, wie der Alzheimer'schen Krankheit, Gehirngefäßverletzungen, Rückgrattraumata oder lateraler amyotrophischer Sklerose.

**Patentansprüche für folgenden Vertragsstaat : ES**

1. Verfahren zur Herstellung von Verbindungen der allgemeinen Formel (I):

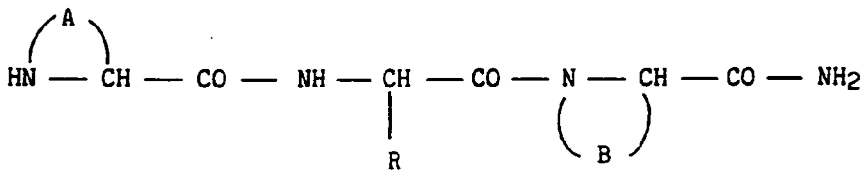 (I)

in der:

A zusammen mit dem Stickstoff- und dem Kohlenstoffatom, an die es gebunden ist:
- eine 2-Oxoperhydroazepin-7-yl-gruppe,
- eine 2-Oxoperhydroazocin-8-yl-gruppe,
- eine 2-Oxoperhydroazonin-9-yl-gruppe,
- eine 2-Oxoperhydroazecin-10-yl-gruppe,
- eine 2-Oxo-2,3,4,7-tetrahydrobenzo[e]azepin-7-yl-gruppe,
- eine 2-Oxo-2,3,6,7-tetrahydrobenzo[d]azepin-7-yl-gruppe,
- eine 2-Oxo-2,5,6,7-tetrahydrobenzo[c]azepin-7-yl-gruppe bedeutet;

B zusammen mit dem Stickstoff- und dem Kohlenstoffatom, an die es gebunden ist, eine polycyclische Struktur bedeutet, die aus den folgenden Strukturen ausgewählt ist:
- 2-Azabicyclo[2.2.1]hept-2,3-ylen,
- 2-Azabicyclo[2.2.2]oct-2,3-ylen, das gegebenenfalls in der 1- und der 4-Stellung durch eine oder zwei geradkettige oder verzweigte $C_1$-$C_4$-Gruppen substituiert ist,
- Perhydroindol-1,2-ylen,
- Perhydroisoindol-2,3-ylen,
- Indol-1,2-ylen,
- Isoindol-2,3-ylen,
- Perhydrochinol-1,2-ylen,
- Perhydroisochinol-2,3-ylen,
- 1,2,3,4-Tetrahydrochinol-1,2-ylen,
- 1,2,3,4-Tetrahydroisochinol-2,3-ylen,
- Cyclopenta[b]pyrrolidin-1,2-ylen,
- Pyrrolidin-1,2-ylen, das gegebenenfalls durch eine oder zwei geradkettige oder verzweigte $C_1$-$C_4$-Alkylgruppen substituiert ist,
- Piperidin-1,2-ylen und
- Thiazolidin-3,4-ylen;

R - ein Wasserstoffatom,
- eine geradkettige oder verzweigte $C_1$-$C_6$-Alkylgruppe, die gegebenenfalls durch eine Aminogruppe oder eine Guanidinogruppe substituiert ist,
- eine (Imidazol-4-yl)-methylgruppe, die gegebenenfalls an einem der Stickstoffatome, durch eine geradkettige oder verzweigte $C_1$-$C_4$-Alkylgruppe substituiert ist,
- eine (Pyrazol-3-yl)-methylgruppe oder
- eine (Pyridin-2-yl)-methylgruppe, die gegebenenfalls durch eine Aminogruppe substituiert ist, bedeutet,

sowie ihrer Enantiomere, Diastereoisomere und Epimere sowie ihrer Additionssalze mit einer pharmazeutisch annehmbaren Säure,

**dadurch gekennzeichnet,** daß man die Aminogruppe einer Aminosäure der Formel (II), die man gegebenenfalls mit Hilfe klassischer Trennmethoden in die Isomeren getrennt hat:

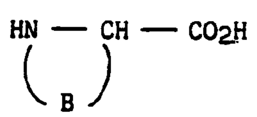 (II)

in der B die für die Formel (I) angegebenen Bedeutungen besitzt, mit einer Schutzgruppe (P), wie der tert.-Butoxycarbonylgruppe (tBOC) oder der Benzyloxycarbonylgruppe (Z) unter Einwirkung eines geeigneten Reagens schützt zur Bildung eines Derivats der Formel (III):

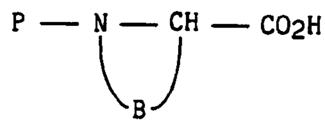

(III)

in der B und P die oben angegebenen Bedeutungen besitzen, welches man bei einer Temperatur zwischen -15 und 0°C in Gegenwart von Triethylamin mit Chlorameisensäureethylester und dann mit Ammoniak umsetzt zur Bildung eines Derivats der Formel (IV):

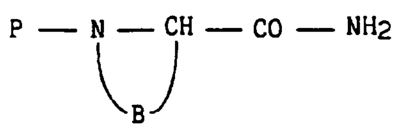

(IV)

in der B und P die oben angegebenen Bedeutungen besitzen,
von dem man die Schutzgruppe mit Hilfe eines geeigneten Verfahrens, beispielsweise durch Einwirkung von gasförmiger Chlorwasserstoffsäure in einem wasserfreien Lösungsmittel, wie Dioxan oder Ethylacetat, in dem Fall, da P = tBOC ist, oder durch Hydrieren dann, wenn P = Z ist, abspaltet zur Bildung eines Derivats der Formel (V):

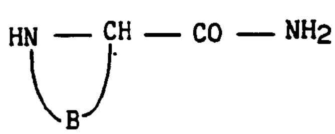

(V)

in der B die bezüglich der Formel (I) angegebenen Bedeutungen besitzt, welches man gewünschtenfalls mit Hilfe klassischer Trennungsmethoden in die Isomeren trennt, und welches man mit einer zweiten geschützten Aminosäure der Formel (VI):

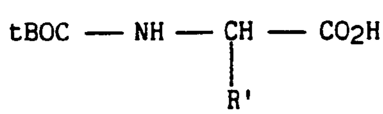

(VI)

in der R' ein Wasserstoffatom, eine geradkettige oder verzeigte $C_1$-$C_6$-Alkylgruppe, die gegebenenfalls durch eine Aminogruppe, die beispielsweise durch eine Benzyloxycarbonylgruppe (Z) geschützt ist, oder eine Guanidinogruppe, die ihrerseits beispielsweise durch eine Nitrogruppe geschützt ist, substituiert ist, eine (Imidazol-4-yl)-methylgruppe, die gegebenenfalls an einem der Stickstoffatome durch eine geradkettige oder verzweigte $C_1$-$C_4$-Alkylgruppe substituiert ist, eine (Pyrazol-3-yl)-methylgruppe, eine (Pyridin-2-yl)-methylgruppe, die gegebenenfalls durch eine Aminogruppe, die beispielsweise durch eine Benzyloxycarbonylgruppe geschützt ist, substituiert ist, bedeutet, kuppelt unter Bildung eines Derivats der Formel (VII):

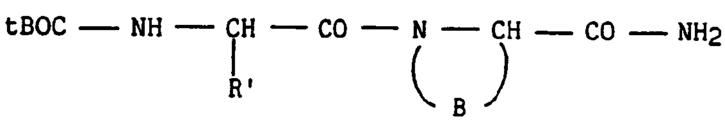

(VII)

in der R' und B die oben angegebenen Bedeutungen besitzen, das man gewünschtenfalls mit Hilfe einer klassischen Trennungsmethode in die Diastereoisomeren oder Enantiomeren auftrennt, von welchen man anschließend durch Einwirkung von gasförmiger Chlorwasserstoffsäure in einem wasserfreien Lösungsmittel, wie beispielsweise Dioxan oder Ethylacetat, die Schutzgruppe abspaltet zur Bildung eines Derivats der Formel (VIII):

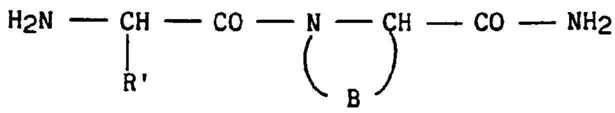

(VIII)

in der R' und B die oben angegebenen Bedeutungen besitzen, welches man mit einer dritten geschützten Aminosäure der Formel (IX):

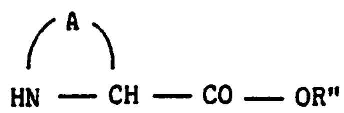

(IX)

in der R" einen Succinimidrest bedeutet, kuppelt, so daß man:

- entweder:
  ein Derivat der Formel (I) erhält, dann, wenn R' von einer geradkettigen oder verzweigten, durch eine geschützte Aminogruppe oder eine geschützte Guanidinogruppe substituierte $C_1$-$C_6$-Alkylgruppe oder von einer durch eine geschützte Aminogruppe substituierte (Pyridin-2-yl)-methylgruppe verschieden ist,
  welches man gewünschtenfalls mit Hilfe einer klassischen Trennungsmethode in die Isomeren auftrennt und welches man erforderlichenfalls in ein Salz mit einer pharmazeutisch annehmbaren Säure überführt,
- oder:
  ein Derivat der Formel (X)

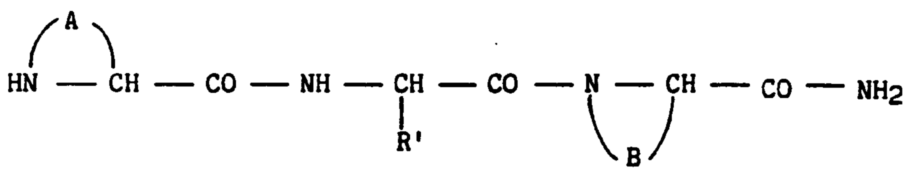

(X)

erhält, dann, wenn A und B die bezüglich der Formel (I) angegebenen Bedeutungen besitzen und R' eine geradkettige oder verzweigte, durch eine geschützte Aminogruppe oder eine geschützte Guanidinogruppe substituierte $C_1$-$C_6$-Alkylgruppe oder R' eine durch eine geschützte Aminogruppe substituierte (Pyridin-2-yl)-methylgruppe bedeutet,

welches man gewünschtenfalls mit Hilfe einer klassischen Trennungsmethode in die Isomeren aufspaltet und von dem man beispielsweise durch katalytische Hydrierung die Schutzgruppe abspaltet, so daß man ein Derivat der Formel (I) erhält, das man gegebenenfalls mit Hilfe einer klassischen Trennungsmethode in die Isomeren aufspaltet und das man erforderlichenfalls in ein Additionssalz mit einer pharmazeutisch annehmbaren Säure überführt.

**2.** Verfahren nach Anspruch 1 zur Herstellung von Verbindungen, bei denen A zusammen mit dem Stickstoffatom und dem Kohlenstoffatom, an die es gebunden ist, einen 2 -Oxoperhydroazepin- 7-yl-Zyklus bildet, ihrer Enantiomeren, Diastereoisomeren und Epimeren sowie ihrer Additionssalze mit einer pharmazeutisch annehmbaren Säure.

**3.** Verfahren nach Anspruch 1 zur Herstellung von Verbindungen, bei denen B zusammen mit dem Stickstoff- und dem Kohlenstoffatom, an die es gebunden ist, einen 2-Azabicyclo-[2.2.1]-hept-2,3-ylen-Zyklus bildet, sowie deren Enantiomere, Diastereoisomere und Epimere sowie deren Additionssalze mit einer pharmazeutisch annehmbaren Säure.

**4.** Verfahren nach Anspruch 1 zur Herstellung von Verbindungen, nämlich AZEP-($N^{\tau}$-Me)-His-ABH-$NH_2$, dessen Enantiomere, Diastereoisomere und Epimere sowie dessen Additionssalze mit einer pharmazeutisch annehmbaren Säure, worin AZEP für den 7-Carbonyl-perhydroazepin-2-on-Rest, ($N^{\tau}$-Me)-His für den 1-Methylhistidylrest und ABH für den 2-Aza-3-carbonylbicyclo[2.2.1]heptan-Rest stehen.

5. Verfahren nach Anspruch 1 zur Herstellung von Verbindungen, nämlich AZEP-(S)-His-(S)-Pro-$NH_2$, dessen Enantiomere, Diastereoisomere und Epimere sowie dessen Additionssalze mit einer pharmazeutisch annehmbaren Säure, worin AZEP für den 7-Carbonyl-perhydroazepin-2-on-Rest, His für den Histidylrest und Pro für den Prolylrest stehen.

**Patentansprüche für folgenden Vertragsstaat : GR**

1. Verfahren zur Herstellung von Verbindungen der allgemeinen Formel (I):

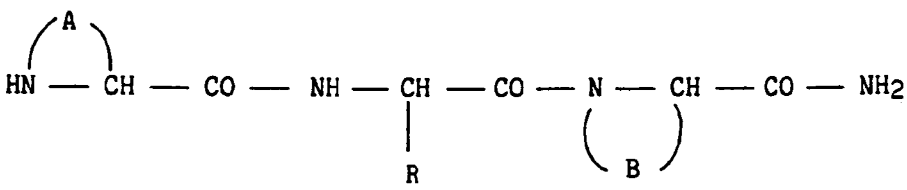

(I)

in der:

A zusammen mit dem Stickstoff- und dem Kohlenstoffatom, an die es gebunden ist:
- eine 2-Oxoperhydroazepin-7-yl-gruppe,
- eine 2-Oxoperhydroazocin-8-yl-gruppe,
- eine 2-Oxoperhydroazonin-9-yl-gruppe,
- eine 2-Oxoperhydroazecin-10-yl-gruppe,
- eine 2-Oxo-2,3,4,7-tetrahydrobenzo[e]azepin-7-yl-gruppe,
- eine 2-Oxo-2,3,6,7-tetrahydrobenzo[d]azepin-7-yl-gruppe,
- eine 2-Oxo-2,5,6,7-tetrahydrobenzo[c]azepin-7-yl-gruppe bedeutet;

B zusammen mit dem Stickstoff- und dem Kohlenstoffatom, an die es gebunden ist, eine polycyclische Struktur bedeutet, die aus den folgenden Strukturen ausgewählt ist:
- 2-Azabicyclo[2.2.1]hept-2,3-ylen,
- 2-Azabicyclo[2.2.2]oct-2,3-ylen, das gegebenenfalls in der 1- und der 4-Stellung durch eine oder zwei geradkettige oder verzweigte $C_1$-$C_4$-Gruppen substituiert ist,
- Perhydroindol-1,2-ylen,
- Perhydroisoindol-2,3-ylen,
- Indol-1,2-ylen,
- Isoindol-2,3-ylen,
- Perhydrochinol-1,2-ylen,
- Perhydroisochinol-2,3-ylen,
- 1,2,3,4-Tetrahydrochinol-1,2-ylen,
- 1,2,3,4-Tetrahydroisochinol-2,3-ylen,
- Cyclopenta[b]pyrrolidin-1,2-ylen,
- Pyrrolidin-1,2-ylen, das gegebenenfalls durch eine oder zwei geradkettige oder verzweigte $C_1$-$C_4$-Alkylgruppen substituiert ist,
- Piperidin-1,2-ylen und
- Thiazolidin-3,4-ylen;

R - ein Wasserstoffatom,
- eine geradkettige oder verzweigte $C_1$-$C_6$-Alkylgruppe, die gegebenenfalls durch eine Aminogruppe oder eine Guanidinogruppe substituiert ist,
- eine (Imidazol-4-yl)-methylgruppe, die gegebenenfalls an einem der Stickstoffatome durch eine geradkettige oder verzweigte $C_1$-$C_4$-Alkylgruppe substituiert ist,
- eine (Pyrazol-3-yl) -methylgruppe oder
- eine (Pyridin-2-yl)-methylgruppe, die gegebenenfalls durch eine Aminogruppe substituiert ist, bedeutet,

sowie ihrer Enantiomere, Diastereoisomere und Epimere sowie ihrer Additionssalze mit einer pharmazeutisch annehmbaren Säure,

**dadurch gekennzeichnet,** daß man die Aminogruppe einer Aminosäure der Formel (II), die man gegebenenfalls mit Hilfe klassischer Trennmethoden in die Isomeren getrennt hat:

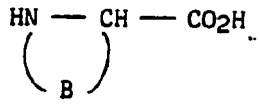

(II)

in der B die für die Formel (I) angegebenen Bedeutungen besitzt, mit einer Schutzgruppe (P), wie der tert.-Butoxycarbonylgruppe (tBOC) oder der Benzyloxycarbonylgruppe (Z) unter Einwirkung eines geeigneten Reagens schützt zur Bildung eines Derivats der Formel (III):

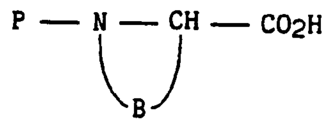

(III)

in der B und P die oben angegebenen Bedeutungen besitzen, welches man bei einer Temperatur zwischen -15 und 0°C in Gegenwart von Triethylamin mit Chlorameisensäureethylester und dann mit Ammoniak umsetzt zur Bildung eines Derivats der Formel (IV):

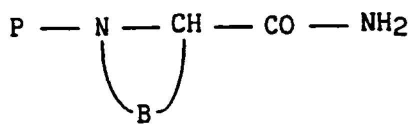

(IV)

in der B und P die oben angegebenen Bedeutungen besitzen,
von dem man die Schutzgruppe mit Hilfe eines geeigneten Verfahrens, beispielsweise durch Einwirkung von gasförmiger Chlorwasserstoffsäure in einem wasserfreien Lösungsmittel, wie Dioxan oder Ethylacetat, in dem Fall, da P = tBOC ist, oder durch Hydrieren dann, wenn P = Z ist, abspaltet zur Bildung eines Derivats der Formel (V):

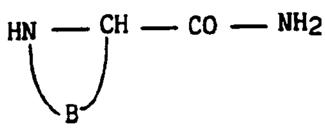

(V)

in der B die bezüglich der Formel (I) angegebenen Bedeutungen besitzt, welches man gewünschtenfalls mit Hilfe klassischer Trennungsmethoden in die Isomeren trennt, und welches man mit einer zweiten geschützten Aminosäure der Formel (VI):

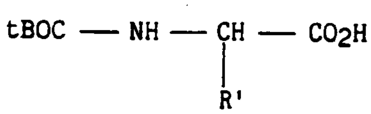

(VI)

in der R' ein Wasserstoffatom, eine geradkettige oder verzeigte $C_1$-$C_6$-Alkylgruppe, die gegebenenfalls durch eine Aminogruppe, die beispielsweise durch eine Benzyloxycarbonylgruppe (Z) geschützt ist, oder eine Guanidinogruppe, die ihrerseits beispielsweise durch eine Nitrogruppe geschützt ist, substituiert ist, eine (Imidazol-4-yl)-methylgruppe, die gegebenenfalls an einem der Stickstoffatome durch eine geradkettige oder verzweigte $C_1$-$C_4$-Alkylgruppe substituiert ist, eine (Pyrazol-3-yl)-methylgruppe, eine (Pyridin-2-yl)-methylgruppe, die gegebenenfalls durch eine Aminogruppe, die beispielsweise durch eine Benzyloxycarbonylgruppe geschützt ist, substituiert ist, bedeutet, kuppelt unter Bildung eines Derivats der Formel (VII):

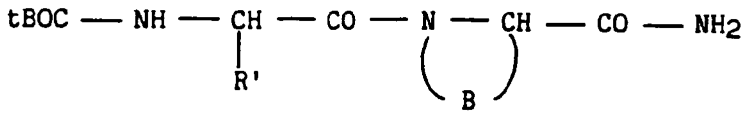

(VII)

in der R' und B die oben angegebenen Bedeutungen besitzen, das man gewünschtenfalls mit Hilfe einer klassischen Trennungsmethode in die Diastereoisomeren oder Enantiomeren auftrennt, von welchen man anschließend durch Einwirkung von gasförmiger Chlorwasserstoffsäure in einem wasserfreien Lösungsmittel, wie beispielsweise Dioxan oder Ethylacetat, die Schutzgruppe abspaltet zur Bildung eines Derivats der Formel (VIII):

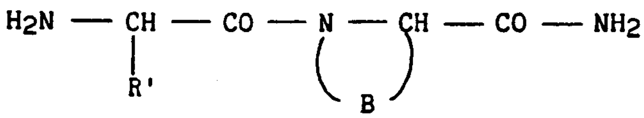

(VIII)

in der R' und B die oben angegebenen Bedeutungen besitzen, welches man mit einer dritten geschützten Aminosäure der Formel (IX):

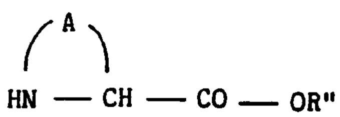

(IX)

in der R" einen Succinimidrest bedeutet, kuppelt, so daß man:

- entweder:
  ein Derivat der Formel (I) erhält, dann, wenn R' von einer geradkettigen oder verzweigten, durch eine geschützte Aminogruppe oder eine geschützte Guanidinogruppe substituierte $C_1$-$C_6$-Alkylgruppe oder von einer durch eine geschützte Aminogruppe substituierte (Pyridin-2-yl)-methylgruppe verschieden ist,
  welches man gewünschtenfalls mit Hilfe einer klassischen Trennungsmethode in die Isomeren auftrennt und welches man erforderlichenfalls in ein Salz mit einer pharmazeutisch annehmbaren Säure überführt,
- oder:
  ein Derivat der Formel (X)

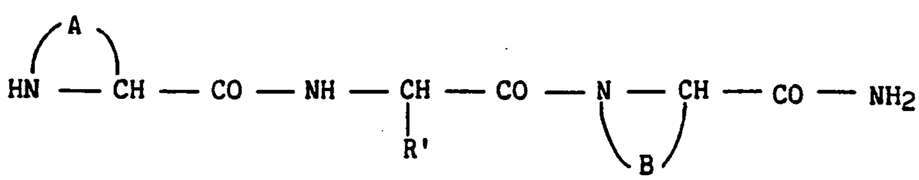

(X)

erhält, dann, wenn A und B die bezüglich der Formel (I) angegebenen Bedeutungen besitzen und R' eine geradkettige oder verzweigte, durch eine geschützte Aminogruppe oder eine geschützte Guanidinogruppe substituierte $C_1$-$C_6$-Alkylgruppe oder R' eine durch eine geschützte Aminogruppe substituierte (Pyridin-2-yl)-methylgruppe bedeutet,

welches man gewünschtenfalls mit Hilfe einer klassischen Trennungsmethode in die Isomeren aufspaltet und von dem man beispielsweise durch katalytische Hydrierung die Schutzgruppe abspaltet, so daß man ein Derivat der Formel (I) erhält, das man gegebenenfalls mit Hilfe einer klassischen Trennungsmethode in die Isomeren aufspaltet und das man erforderlichenfalls in ein Additionssalz mit einer pharmazeutisch annehmbaren Säure überführt.

2. Verfahren nach Anspruch 1 zur Herstellung von Verbindungen, bei denen A zusammen mit dem Stickstoffatom und dem Kohlenstoffatom, an die es gebunden ist, einen 2-Oxoperhydroazepin-7-yl-Zyklus bildet, ihrer Enantiomeren, Diastereoisomeren und Epimeren sowie ihrer Additionssalze mit einer pharmazeutisch annehmbaren Säure.

3. Verfahren nach Anspruch 1 zur Herstellung von Verbindungen, bei denen B zusammen mit dem Stickstoff- und dem Kohlenstoffatom, an die es gebunden ist, einen 2-Azabicyclo-[2.2.1]-hept-2,3-ylen-Zyklus bildet, sowie deren Enantiomere, Diastereoisomere und Epimere sowie deren Additionssalze mit einer pharmazeutisch annehmbaren Säure.

4. Verfahren nach Anspruch 1 zur Herstellung von Verbindungen, nämlich AZEP-($N^{\tau}$-Me)-His-ABH-$NH_2$, dessen Enantiomere, Diastereoisomere und Epimere sowie dessen Additionssalze mit einer pharmazeutisch annehmbaren Säure, worin AZEP für den 7-Carbonyl-perhydroazepin-2-on-Rest, ($N^{\tau}$-Me)-His für den 1-Methylhistidylrest und ABH für den 2-Aza-3-carbonylbicyclo[2.2.1]heptan-Rest stehen.

5. Verfahren nach Anspruch 1 zur Herstellung von Verbindungen, nämlich AZEP-(S)-His-(S)-Pro-$NH_2$, dessen Enantiomere, Diastereoisomere und Epimere sowie dessen Additionssalze mit einer pharmazeutisch annehmbaren Säure, worin AZEP für den 7-Carbonyl-perhydroazepin-2-on-Rest, His für den Histidylrest und Pro für den Prolylrest stehen.